(19) Europäisches Patentamt
European Patent Office
Office européen des brevets

(11) **EP 4 389 153 A1**

(12) **EUROPEAN PATENT APPLICATION**
published in accordance with Art. 153(4) EPC


(43) Date of publication:
**26.06.2024 Bulletin 2024/26**

(21) Application number: **22869373.5**

(22) Date of filing: **16.09.2022**

(51) International Patent Classification (IPC):
***A61K 47/68*** *(2017.01)* ***C07K 16/28*** *(2006.01)*
***C07D 307/20*** *(2006.01)* ***A61P 35/00*** *(2006.01)*

(52) Cooperative Patent Classification (CPC):
**A61K 47/68; A61P 35/00; C07D 307/20; C07K 16/28**

(86) International application number:
**PCT/CN2022/119214**

(87) International publication number:
**WO 2023/041006 (23.03.2023 Gazette 2023/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.09.2021 CN 202111085308**



(71) Applicant: **CHIA TAI TIANQING PHARMACEUTICAL GROUP CO., LTD.**
**Lianyungang,**
**Jiangsu 222062 (CN)**

(72) Inventors:
- **CHEN, Tianxi**
**Nanjing, Jiangsu 211100 (CN)**
- **XU, Tongjie**
**Nanjing, Jiangsu 211100 (CN)**
- **TANG, Xiaoqi**
**Nanjing, Jiangsu 211100 (CN)**
- **FENG, Weiwei**
**Nanjing, Jiangsu 211100 (CN)**
- **ZHANG, Zhengping**
**Nanjing, Jiangsu 211100 (CN)**

(74) Representative: **Müller Schupfner & Partner Patent- und Rechtsanwaltspartnerschaft mbB (Muc)**
**Bavariaring 11**
**80336 München (DE)**


Remarks:
The complete document including Reference Table(s) and the Sequence Listing(s) can be downloaded from the EPO website

(54) **ANTI-HER3 ANTIBODY DRUG CONJUGATE, COMPOSITION THEREOF, AND USE THEREOF**


(57) Provided is an anti-HER3 antibody drug conjugate, specifically comprising an antibody moiety, an intermediate linker moiety, and a cytotoxic drug moiety which are linked together. The provided antibody drug conjugate achieves excellent anti-tumor activity or/and better safety. The provided antibody drug conjugate can be used for the treatment of cancer.


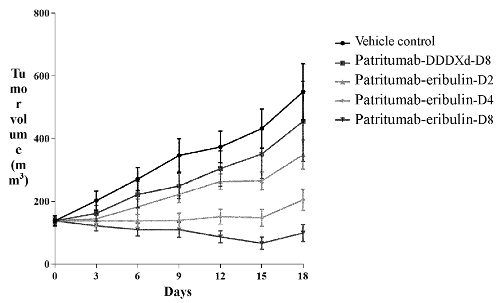


FIG. 4

EP 4 389 153 A1

## Description

### TECHNICAL FIELD

The present disclosure relates to an antibody-drug conjugate comprising an antibody moiety, an intermediate linker moiety, and a cytotoxic drug moiety that are linked. The present disclosure further relates to use of the antibody-drug conjugate for preparing a medicament for treating cancer.

### BACKGROUND

HER3 (human epidermal growth factor receptor 3), also known as ErbB-3 (receptor tyrosine-protein kinase erbB-3), is a member of the human epidermal growth factor receptor (HER/EGFR/ERBB) family, which includes EGFR (ErbB-1), HER2/c-neu (ErbB-2), HER3 (ErbB-3) and HER4 (ErbB-4). HER3 has an extracellular ligand binding domain (ECD), a dimerization domain within the ECD, a transmembrane domain, a protein tyrosine kinase domain (TKD), and a C-terminal phosphorylation domain, but it lacks intracellular tyrosine kinase activity. The ligand of HER3, neuregulin (NRG, also known as heregulin (HRG)), binds to the extracellular domain of HER3 and activates receptor-mediated signaling pathways by promoting dimerization with other HER family members and transphosphorylation of its intracellular domain. Dimers formed by HER3 and other members of the HER family expand the signaling potential of HER3 and serve not only as a means of signal diversification, but also as a means of signal amplification. For example, the HER2/HER3 heterodimer induces one of the most important mitogenic signals in HER family members. HER3 is ubiquitously expressed in a variety of cancers, including breast cancer, ovarian cancer, colon cancer, gastric cancer, lung cancer, skin cancer, and pancreatic cancer.

Antibody-drug conjugates (ADCs) are a class of drugs that combine the high specificity of therapeutic antibodies and the high killing activity of cytotoxic drugs, where the therapeutic antibody moiety is linked to the cytotoxic drug moiety via an intermediate linker moiety. Currently, at least ten ADC drugs are marketed globally. Among these drugs, antibody moieties of brentuximab vedotin, polatuzumab vedotin, and enfortumab vedotin are directed against targets CD30, CD79b, and Nectin-4, respectively, antibody moieties of trastuzumab emtansine and trastuzumab deruxtecan are directed against target HER2, antibody moieties of gemtuzumab ozogamicin and inotuzumab ozogamicin are directed against targets CD33 and CD22, respectively, antibody moiety of sacituzumab govitecan is directed against target TROP2, and the newly approved belantamab mafodotin and loncastuximab tesirine are directed against targets BCMA and CD19, respectively. For the cytotoxic drug moieties, auristatins acting on microtubules are adopted for brentuximab vedotin, polatuzumab vedotin, enfortumab vedotin, and belantamab mafodotin, maytansinoid toxin molecules acting on microtubules are adopted for trastuzumab emtansine, calicheamicin toxin molecules acting on DNA are adopted for gemtuzumab ozogamicin and inotuzumab ozogamicin, camptothecin toxin molecules are adopted for trastuzumab deruxtecan and sacituzumab govitecan, and PBD dimers acting on DNA are adopted for loncastuximab tesirine. For the intermediate linker moiety, a non-cleavable linker is adopted for trastuzumab emtansine and belantamab mafodotin, while a cleavable linker is adopted for the remaining eight molecules.

Eribulin (formula I below) is a synthetic analog of the natural marine product halichondrin B. It can inhibit microtubule growth phase and acts through a tubulin-based antimitotic mechanism, resulting in arrest of the G2/M cell cycle, disruption of the mitotic spindle, and finally apoptosis after long-term mitotic arrest. Eribulin is currently approved for the treatment of metastatic breast cancer and soft tissue sarcoma.

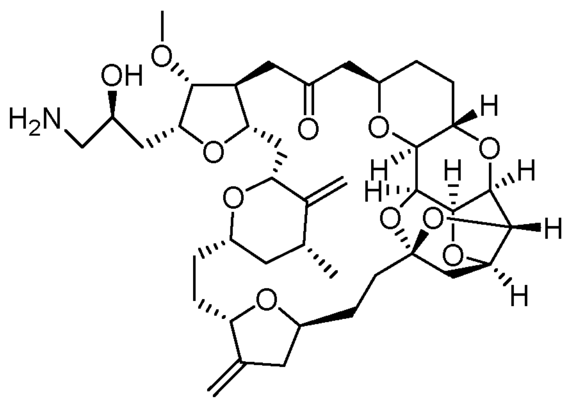

I

ADC drugs combine the dual advantages of high potency of cytotoxic small molecules and high selectivity of antibodies to specific tumor cells; however, there is still a need to develop highly potent and low-toxic ADC drugs that

can target more indications.

## SUMMARY

*Antibody-Drug Conjugate (ADC)*

The present disclosure provides an antibody-drug conjugate, wherein an antibody or an antigen-binding fragment thereof is conjugated to a cytotoxic drug eribulin or a derivative thereof; preferably, the antibody or the antigen-binding fragment thereof specifically binds to HER3.

In one aspect, the present disclosure provides an antibody-drug conjugate of general formula Ab-$(L\text{-}U)_n$ or a pharmaceutically acceptable salt or a solvate thereof, wherein Ab represents an antibody moiety, L represents a linker moiety, U represents a cytotoxic drug moiety, and n is an integer or a decimal selected from numbers from 1 to 10. In certain embodiments, the antibody moiety Ab is linked to the linker moiety via a specific functional group, and the antibody moiety can specifically bind to an antigen.

In one aspect, the present disclosure provides an antibody-drug conjugate of general formula Ab-$(L\text{-}U)_n$ or a pharmaceutically acceptable salt or a solvate thereof, wherein a cytotoxic drug moiety U is conjugated to an antibody moiety Ab via a linker moiety L. In some specific embodiments, in the antibody-drug conjugate or the pharmaceutically acceptable salt or the solvate thereof provided herein, each cytotoxic drug moiety U is conjugated to the antibody moiety Ab via one linker moiety L. The linker moiety L disclosed herein may be linked to the antibody moiety by any method known in the art; preferably, the linker moiety is linked to the antibody moiety via sulfydryl and/or amino. In some more preferred embodiments, the linker moiety disclosed herein is linked to the antibody moiety via sulfydryl.

In one aspect, the present disclosure provides an antibody-drug conjugate of general formula Ab-$(L\text{-}U)_n$ or a pharmaceutically acceptable salt or a solvate thereof, wherein a cytotoxic drug moiety U is conjugated to an antibody moiety Ab via a linker moiety L, and the linker moiety may be a cleavable linker or a non-cleavable linker. In some embodiments, the linker moiety disclosed herein is a cleavable linker, which may be, for example, a low pH-dependent degradation type (including a hydrazone bond, a carbonate bond, and the like), a proteolytic type (including a peptide-based bond), a high glutathione concentration-dependent degradation type (including a disulfide bond), or the like. The cleavable linker may be cleaved within the target cell, thereby releasing the cytotoxic drug. In other embodiments, the linker moiety disclosed herein is a non-cleavable linker, which may be, for example, maleimidocaproyl or the like.

In one aspect, the present disclosure provides an antibody-drug conjugate of general formula Ab-$(L\text{-}U)_n$ or a pharmaceutically acceptable salt or a solvate thereof, wherein an antibody moiety Ab is conjugated to one or more cytotoxic drug moieties U; the cytotoxic drug may be selected from, e.g., alkaloids, antimetabolites, anti-tumor antibiotics, alkylating agents, platinum-based drugs, and the like, and preferably, the cytotoxic drug is a microtubule inhibitor cytotoxic drug (including maytansinoid, auristatin, eribulin, and the like) or a cytotoxic drug acting on DNA (including calicheamicin, duocarmycin, PBD (pyrrolobenzodiazepine), a topoisomerase I inhibitor, and the like).

In some specific embodiments, the cytotoxic drug moiety U of the antibody-drug conjugate of general formula Ab-$(L\text{-}U)_n$ or the pharmaceutically acceptable salt or the solvate thereof provided herein is a microtubule inhibitor.

In some specific embodiments, the cytotoxic drug moiety U of the antibody-drug conjugate of general formula Ab-$(L\text{-}U)_n$ or the pharmaceutically acceptable salt or the solvate thereof provided herein is eribulin or a derivative thereof.

In certain embodiments, the cytotoxic drug moiety is linked to the linker moiety via a functional group, so that cytotoxic drug molecules can be liberated in tumor cells to exert an anti-tumor effect.

In one aspect, the present disclosure provides an antibody-drug conjugate of general formula Ab-$(L\text{-}U)_n$ or a pharmaceutically acceptable salt or a solvate thereof, wherein Ab represents an antibody moiety, L represents a linker moiety, U represents a cytotoxic drug moiety, and n is an integer or a decimal selected from numbers from 1 to 10, wherein the antibody-drug conjugate comprises a structure of formula IIa below:

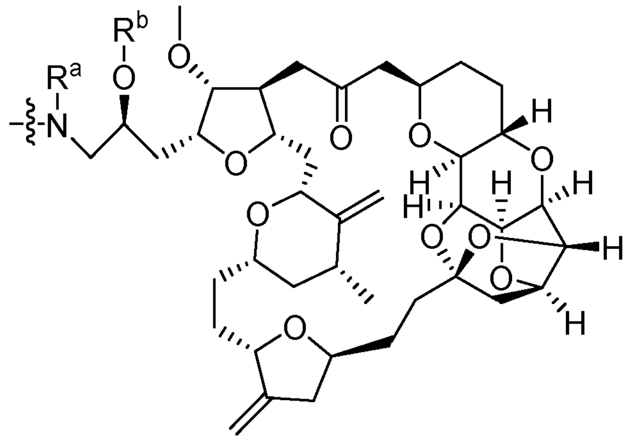

IIa,

wherein,

$R^a$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;
$R^b$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;
or,
$R^a$ and $R^b$, together with an atom to which they are attached, form optionally substituted 5-8 membered heterocyclyl. In some embodiments, $R^a$ and $R^b$ are each independently selected from a hydrogen atom, methyl, ethyl, propyl, and isopropyl. In some embodiments, $R^a$ and $R^b$ are hydrogen atoms.

In some embodiments, the antibody-drug conjugate comprises a structure of formula IIa-1 described below:

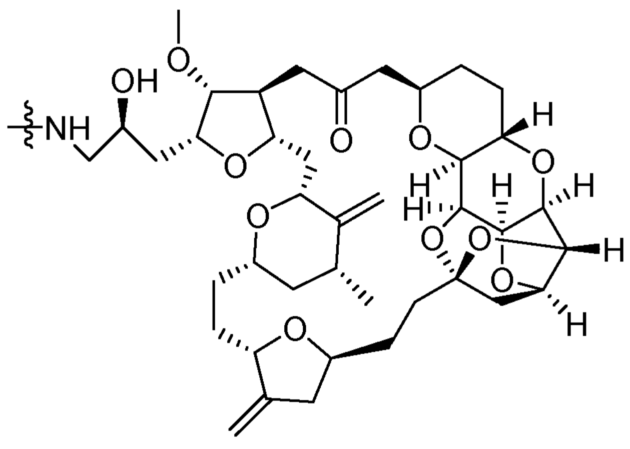

IIa-1.

In some embodiments, the present disclosure provides an antibody-drug conjugate of general formula Ab-(L-U)$_n$ or a pharmaceutically acceptable salt or a solvate thereof, wherein Ab represents an antibody moiety, L represents a linker moiety, U represents a cytotoxic drug moiety, and n is an integer or a decimal selected from numbers from 1 to 10, wherein -U is a structure of formula IIa:
wherein,

$R^a$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;
$R^b$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$

heteroaryl;

or,

$R^a$ and $R^b$, together with an atom to which they are attached, form optionally substituted 5-8 membered heterocyclyl. In some embodiments, $R^a$ and $R^b$ are each independently selected from a hydrogen atom, methyl, ethyl, propyl, and isopropyl. In some embodiments, $R^a$ and $R^b$ are hydrogen atoms. In some embodiments, -U is a structure of formula IIa-1.

In some embodiments, the antibody-drug conjugate of general formula Ab-(L-U)$_n$ or the pharmaceutically acceptable salt or the solvate thereof provided herein comprises a structure of formula IIIa below:

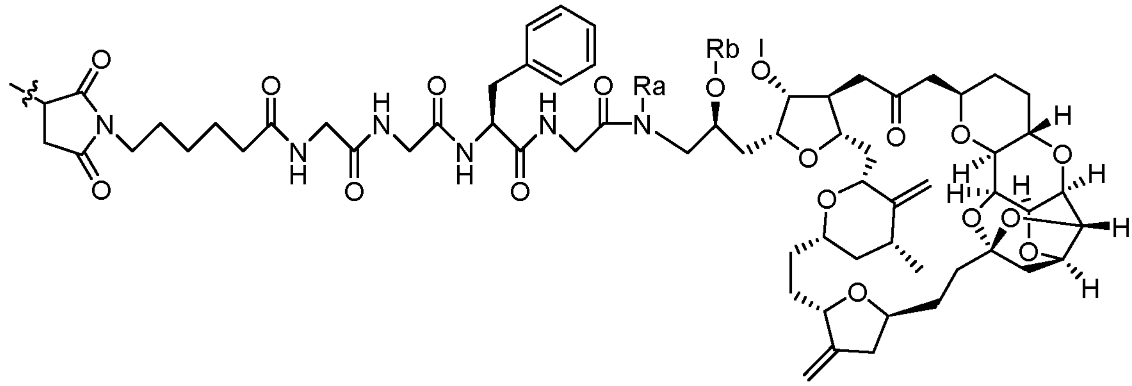

IIIa,

wherein,

$R^a$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;

$R^b$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;

or,

$R^a$ and $R^b$, together with an atom to which they are attached, form optionally substituted 5-8 membered heterocyclyl. In some embodiments, $R^a$ and $R^b$ are each independently selected from a hydrogen atom, methyl, ethyl, propyl, and isopropyl. In some embodiments, $R^a$ and $R^b$ are hydrogen atoms.

In some embodiments, the antibody-drug conjugate comprises a structure of formula IIIa-1 described below:

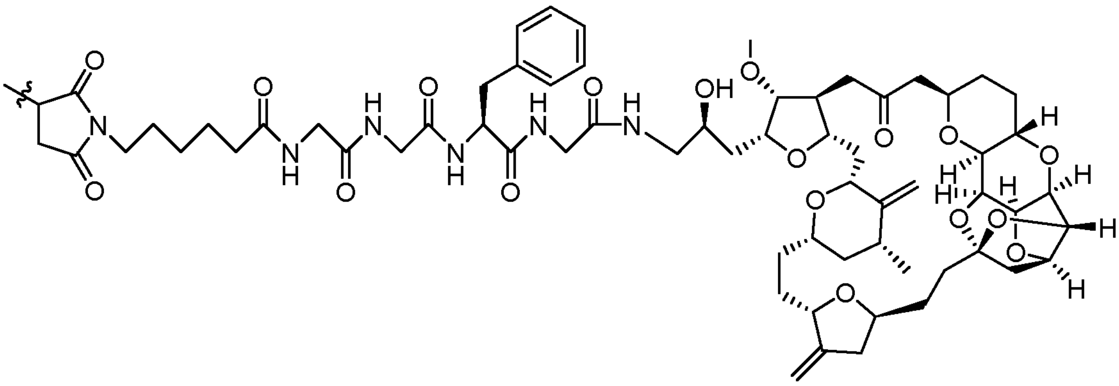

IIIa-1.

In some embodiments, the antibody-drug conjugate of general formula Ab-(L-U)$_n$ or the pharmaceutically acceptable salt or the solvate thereof provided herein is a structure of formula IV below:

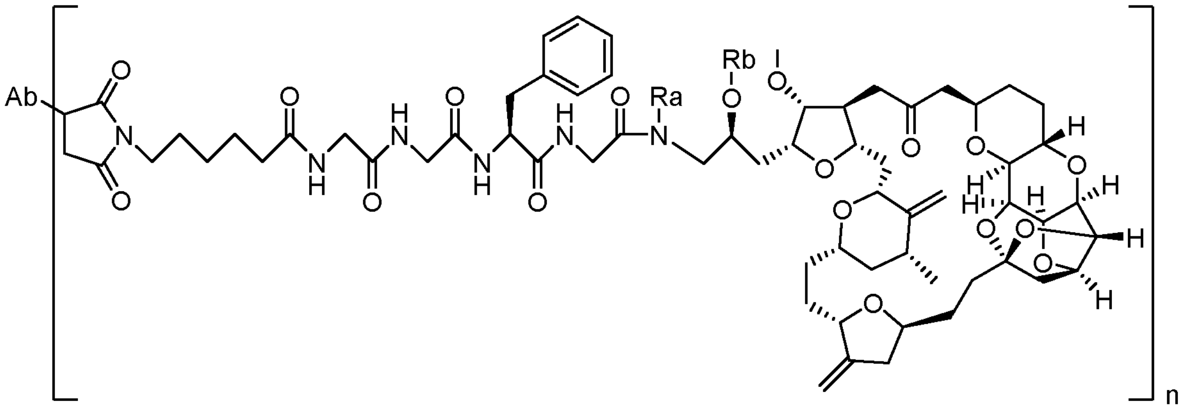

IV,

wherein, Ab represents an antibody moiety;
n is an integer or a decimal selected from numbers from 1 to 10;
$R^a$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;
$R^b$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;
or,
$R^a$ and $R^b$, together with an atom to which they are attached, form optionally substituted 5-8 membered heterocyclyl. In some embodiments, $R^a$ and $R^b$ are each independently selected from a hydrogen atom and $C_{1-5}$ alkyl (preferably $C_{1-4}$ alkyl, e.g., $C_{1-3}$ alkyl). In some embodiments, $R^a$ and $R^b$ are each independently selected from a hydrogen atom, methyl, ethyl, propyl, and isopropyl. In one embodiment, $R^a$ and $R^b$ are hydrogen atoms. In one specific embodiment, the present disclosure provides an antibody-drug conjugate of formula IV-1 below or a pharmaceutically acceptable salt or a solvate thereof,

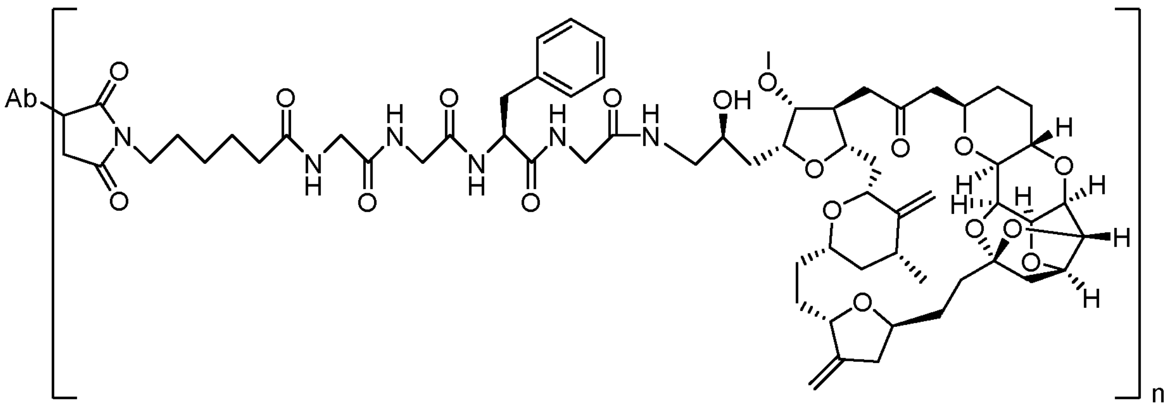

IV-1,

wherein,

Ab is an antibody moiety, and
n is an integer or a decimal selected from numbers from 1 to 10.

In some embodiments, in the antibody-drug conjugate or the pharmaceutically acceptable salt or the solvate thereof described above, n is 2-4.8, 2.6-4.8, 3.5-4.8, 4-4.8, 2-4.5, 2.6-4.5, 3.5-4.5, 4-4.5, 3.5-4.2, 3.5-4, 4-4.2, 7-8, 7-7.9, 7-7.6, 7-7.5, 7.1-8, 7.1-7.9, 7.1-7.6, 7.5-8, 7.6-8, or 7.6-7.9. In some embodiments, n is about 2.6, about 4, about 4.2, about 4.8, about 7, about 7.1, about 7.5, about 7.6, about 7.9, or about 8.

The number of cytotoxic drugs conjugated to the antibody moiety in the antibody-drug conjugate (ADC) disclosed herein may vary such that the antibody-drug conjugate or the pharmaceutically acceptable salt or the solvate thereof provided herein may be heterogeneous, i.e., the antibody-drug conjugate or the pharmaceutically acceptable salt or the

solvate thereof disclosed herein includes antibodies or antigen-binding fragments thereof to which different numbers of cytotoxic drugs are conjugated; for example, 0 (i.e., no cytotoxic drug), 1, 2, 3, 4, 5, 6, 7, 8 or more cytotoxic drug molecules are conjugated to 1 antibody or antigen-binding fragment molecule.

By controlling the ratio of the antibody or the antigen-binding fragment thereof to which different numbers of cytotoxic drugs are conjugated described above, antibody-drug conjugates having different drug antibody ratios (DARs) or pharmaceutically acceptable salts or solvates thereof can be produced. "DAR" and "n" are used interchangeably herein. It will be understood that the DAR or n is the average molar ratio of the cytotoxic drug to the antibody or the antigen-binding fragment thereof in the ADC, i.e., the average number of cytotoxic drugs conjugated per antibody or antigen-binding fragment molecule. For example, "DAR of about 4" or "n of about 4" refers to an antibody-drug conjugate or a pharmaceutically acceptable salt or a solvate thereof as follows: a heterogeneous mixture containing each molecule of antibody or antigen-binding fragment thereof to which varying amounts of cytotoxic drugs conjugated (e.g., 0, 1, 2, 3, 4, 5, 6, 7 or 8 cytotoxic drugs are conjugated to each antibody or an antigen-binding fragment thereof) but the average molar ratio of cytotoxic drugs to the antibody or the antigen-binding fragment thereof is about 4. Similarly, "DAR of about 8" or "n of about 8" means that the average molar ratio of cytotoxic drugs to the antibody or the antigen-binding fragment thereof in the ADC is about 8.

In one aspect, the present disclosure provides an antibody-drug conjugate having the general formula Ab-(L-U)$_n$ or a pharmaceutically acceptable salt or a solvate thereof, wherein Ab (antibody moiety) can specifically bind to a tumor antigen that can be selected from any tumor treatment target known in the art, and non-limiting examples of the target include HER2, HER3, EGFR, CD20, CD30, CD33, CD47, CD79b, VEGF, VEGFR, MET, RET, PD-1, or PD-L1. In some embodiments, the present disclosure provides an antibody-drug conjugate of formula IV-1 or a pharmaceutically acceptable salt or a solvate thereof, wherein Ab (antibody moiety) can specifically bind to a tumor antigen that can be selected from any tumor treatment target known in the art, and non-limiting examples of the target include HER2, HER3, EGFR, CD20, CD30, CD33, CD47, CD79b, VEGF, VEGFR, MET, RET, PD-1, or PD-L1.

In some embodiments, in the antibody-drug conjugate or the pharmaceutically acceptable salt or the solvate thereof, Ab is an anti-HER3 antibody or an antigen-binding fragment thereof.

In some embodiments, Ab is an anti-HER3 antibody or an antigen-binding fragment thereof, wherein the anti-HER3 antibody or the antigen-binding fragment thereof comprises heavy chain CDR (HCDR)1 comprising an amino acid sequence set forth in SEQ ID NO: 1, HCDR2 comprising an amino acid sequence set forth in SEQ ID NO: 2, HCDR3 comprising an amino acid sequence set forth in SEQ ID NO: 3, light chain CDR (LCDR)1 comprising an amino acid sequence set forth in SEQ ID NO: 4, LCDR2 comprising an amino acid sequence set forth in SEQ ID NO: 5, and LCDR3 comprising an amino acid sequence set forth in SEQ ID NO: 6.

The amino acid sequences of CDRs of the anti-HER3 antibody or the antigen-binding fragment thereof are provided in Table S 1 below.

In some embodiments, the antibody moiety of the antibody-drug conjugate of general formula Ab-(L-U)$_n$ or the pharmaceutically acceptable salt or the solvate thereof provided herein is patritumab, which has the sequence shown in Table S1 below.

Table S 1. Amino acid sequences of CDRs and variable regions of patritumab

| Name | Amino acid sequence | SEQ ID NO: |
|---|---|---|
| HCDR1 | GGSFSGY | 1 |
| HCDR2 | NHSGS | 2 |
| HCDR3 | DKWTWYFDL | 3 |
| LCDR1 | QSVLYSSSNRNYLA | 4 |
| LCDR2 | WASTRES | 5 |
| LCDR3 | QQYYSTPRT | 6 |
| Heavy chain variable region | QVQLQQWGAGLLKPSETLSLTCAVY GGSFSGYYWSWIRQPPGKGLEWIGEI NHSGSTNYNPSLKSRVTISVETSKNQF SLKLSSVTAADTAVYYCARDKWTWY FDLWGRGTLVTVSS | 7 |

(continued)

| Name | Amino acid sequence | SEQ ID NO: |
|---|---|---|
| Light chain variable region | DIEMTQSPDSLAVSLGERATINCRSSQ SVLYSSSNRNYLAWYQQNPGQPPKL LIYWASTRESGVPDRFSGSGSGTDFTL TISSLQAEDVAVYYCQQYYSTPRTFG QGTKVEIK | 8 |

In some embodiments, the anti-HER3 antibody or the antigen-binding fragment thereof comprises a heavy chain variable region and a light chain variable region, wherein the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 7, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 8. In some embodiments, the anti-HER3 antibody or the antigen-binding fragment thereof comprises a heavy chain variable region and a light chain variable region, wherein the heavy chain variable region comprises the amino acid sequence set forth in SEQ ID NO: 7, and the light chain variable region comprises the amino acid sequence set forth in SEQ ID NO: 8. In some embodiments, the amino acid sequence of the heavy chain variable region of the anti-HER3 antibody or the antigen-binding fragment thereof is set forth in SEQ ID NO: 7, and the amino acid sequence of the light chain variable region of the anti-HER3 antibody or the antigen-binding fragment thereof is set forth in SEQ ID NO: 8.

In some embodiments, the anti-HER3 antibody or the antigen-binding fragment thereof may further comprise a constant region of an immunoglobulin, or a fragment, an analog, a variant, or a derivative of the constant region. In some embodiments, the constant region is derived from a human immunoglobulin heavy chain, e.g., a heavy chain of IgG1, IgG2, IgG3 and IgG4, or other types of immunoglobulins, preferably a heavy chain of IgG1. In some embodiments, the constant region may comprise modifications described in context, e.g., insertion, deletion, substitution, or chemical modification of amino acids. In some embodiments, the constant region comprises a mutation that alters effector function. In some embodiments, any amino acid residue of the constant region may be substituted by an amino acid residue of any allotype.

In some embodiments, the anti-HER3 antibody or the antigen-binding fragment thereof comprises a heavy chain and a light chain, wherein the heavy chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 9, and the light chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to an amino acid sequence set forth in SEQ ID NO: 10. In some embodiments, the anti-HER3 antibody or the antigen-binding fragment thereof comprises a heavy chain and a light chain, wherein the heavy chain comprises the amino acid sequence set forth in SEQ ID NO: 9, and the light chain comprises the amino acid sequence set forth in SEQ ID NO: 10. In some embodiments, the amino acid sequence of the heavy chain of the anti-HER3 antibody or the antigen-binding fragment thereof is set forth in SEQ ID NO: 9, and the amino acid sequence of the light chain of the anti-HER3 antibody or the antigen-binding fragment thereof is set forth in SEQ ID NO: 10.

QVQLQQWGAGLLKPSETLSLTCAVYGGSFSGYYWSWIRQPPGKGLEWIGEINHSGSTNYNPSLKSRVTIS
VETSKNQFSLKLSSVTAADTAVYYCARDKWTWYFDLWGRGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNT
KVDKRVEPKSCDKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPGK (SEQ ID NO: 9);

DIEMTQSPDSLAVSLGERATINCRSSQSVLYSSSNRNYLAWYQQNPGQPPKLLIYWASTRESGVPDRFSG
SGSGTDFTLTISSLQAEDVAVYYCQQYYSTPRTFGQGTKVEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQGLSSP
VTKSFNRGEC (SEQ ID NO: 10).

In some embodiments, the anti-HER3 antibody is selected from the group consisting of patritumab, seribantumab, elgemtumab, duligotuzumab, CDX-3379, lumretuzumab, and GSK2849330. In some specific embodiments, the anti-HER3 antibody is patritumab.

In some embodiments, the anti-HER3 antibody or the antigen-binding fragment thereof is selected from a monoclonal antibody, a multispecific antibody, a Fab fragment, a Fab' fragment, a F(ab)'2 fragment, an Fd fragment, an Fv fragment, a dAb fragment, an isolated CDR region, a scFv, a nanobody, and a fusion protein.

In some embodiments, the general formula of the antibody-drug conjugate or the pharmaceutically acceptable salt or the solvate thereof disclosed herein is Ab-(L-U)$_n$, wherein Ab is modifiable, e.g., comprising one or more changes, additions, or deletions of the amino acid sequence. Herein, the modified Ab still retains the activity of specifically binding to its corresponding antigen.

In some embodiments, the antibody-drug conjugate or the pharmaceutically acceptable salt or the solvate thereof provided herein is a structure shown below:

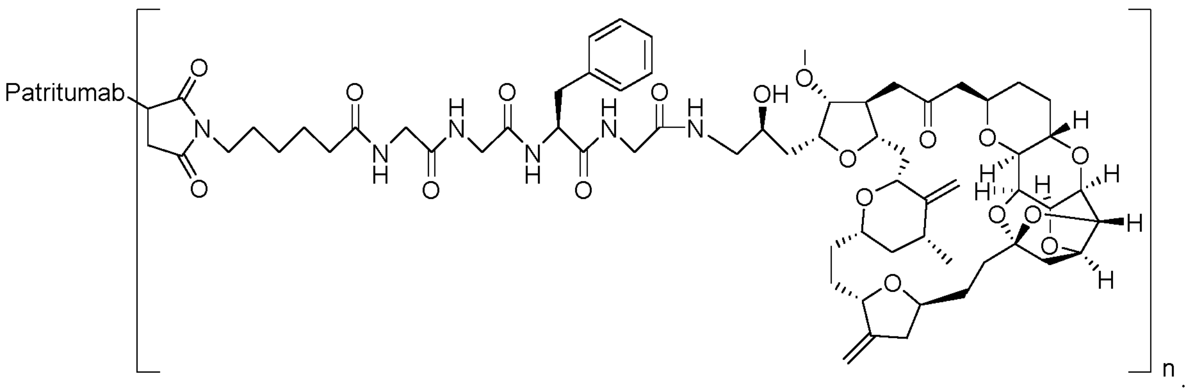

In some such embodiments, n is 2-4.8, 2.6-4.8, 3.5-4.8, 4-4.8, 2-4.5, 2.6-4.5, 3.5-4.5, 4-4.5, 3.5-4.2, 3.5-4, 4-4.2, 7-8, 7-7.9, 7-7.6, 7-7.5, 7.1-8, 7.1-7.9, 7.1-7.6, 7.5-8, 7.6-8, or 7.6-7.9. In some specific embodiments, n is about 2.6, about 4, about 4.2, about 4.8, about 7, about 7.1, about 7.5, about 7.6, about 7.9, or about 8.

In one aspect, the antibody-drug conjugate having the general formula Ab-(L-U)$_n$ or the pharmaceutically acceptable salt or the solvate thereof provided herein exhibits one of or a combination of several of the following properties:

(a) binding to HER3;
(b) blocking binding of HER3 to a ligand;
(c) showing endocytosis in cells expressing HER3;
(d) having killing activity against HER3-expressing tumor cells;
(e) having bystander effect.

In some embodiments, the antibody-drug conjugate or the pharmaceutically acceptable salt or the solvate thereof binds to human HER3.

In some embodiments, the antibody-drug conjugate or the pharmaceutically acceptable salt or the solvate thereof provided herein shows strong endocytosis in cells with different HER3 expression amounts and can continuously accumulate endocytosed ADC amount with increasing endocytosis time.

*Pharmaceutical Composition*

In one aspect, the present disclosure provides a pharmaceutical composition comprising the antibody-drug conjugate or the pharmaceutically acceptable salt or the solvate thereof disclosed herein. In some embodiments, the present disclosure provides a pharmaceutical composition comprising the antibody-drug conjugate or the pharmaceutically acceptable salt or the solvate thereof according to the present disclosure and a pharmaceutically acceptable

carrier. The pharmaceutically acceptable carriers include, for example, excipients, diluents, encapsulating materials, fillers, buffering agents, or other agents.

*Use*

In one aspect, the present disclosure provides use of the antibody-drug conjugate or the pharmaceutically acceptable salt or the solvate thereof disclosed herein for preparing a medicament for treating cancer. In one aspect, the present disclosure provides use of a pharmaceutical composition comprising the antibody-drug conjugate or the pharmaceutically acceptable salt or the solvate thereof disclosed herein and a pharmaceutically acceptable carrier for preparing a medicament for treating cancer. In one aspect, the present disclosure provides use of the pharmaceutical composition disclosed herein for preparing a medicament for treating cancer.

In one aspect, the present disclosure provides the antibody-drug conjugate or the pharmaceutically acceptable salt or the solvate thereof or the pharmaceutical composition described above for use in treating cancer.

In one aspect, the present disclosure provides a method for treating cancer, which comprises administering to a patient in need thereof a therapeutically effective amount of the antibody-drug conjugate or the pharmaceutically acceptable salt or the solvate thereof disclosed herein or a pharmaceutical composition comprising the antibody-drug conjugate or the pharmaceutically acceptable salt or the solvate thereof disclosed herein and a pharmaceutically acceptable carrier. In one aspect, the present disclosure provides a method for treating cancer, which comprises administering to a patient in need thereof a therapeutically effective amount of the pharmaceutical composition disclosed herein. In some embodiments, the method comprises contacting tumor cells with the antibody-drug conjugate or the pharmaceutically acceptable salt or the solvate thereof or the pharmaceutical composition, thereby killing the tumor cells or inhibiting growth of the tumor cells.

In some embodiments, administration of a therapeutically effective amount of the antibody-drug conjugate or the pharmaceutically acceptable salt or the solvate thereof disclosed herein or the pharmaceutical composition disclosed herein to a patient can kill tumor cells or inhibit growth of the tumor cells.

In one aspect, the present disclosure provides use of the antibody-drug conjugate or the pharmaceutically acceptable salt or the solvate thereof disclosed herein in treating cancer. In one aspect, the present disclosure provides use of the pharmaceutical composition comprising the antibody-drug conjugate or the pharmaceutically acceptable salt or the solvate thereof disclosed herein and a pharmaceutically acceptable carrier in treating cancer. In one aspect, the present disclosure also provides use of the pharmaceutical composition disclosed herein in treating cancer.

In some embodiments, in the methods or uses described above, the patient is not suitable for treatment with drugs targeting HER2. In some embodiments, in the methods or uses described above, the patient is resistant to drugs targeting HER2.

In some embodiments, in the above methods or uses, the cancer is HER3-positive cancer. In some embodiments, the antibody-drug conjugate or the pharmaceutically acceptable salt or the solvate thereof disclosed herein can be used to treat cancer expressing the HER3 protein. In some embodiments, administration of a therapeutically effective amount of the antibody-drug conjugate or the pharmaceutically acceptable salt or the solvate thereof disclosed herein or the pharmaceutical composition disclosed herein to a patient can kill HER3-expression tumor cells or inhibit growth of the HER3-expression tumor cells. Examples of cancer include, but are not limited to, biliary tract cancer, carcinosarcoma, esophageal cancer, esophagogastric junction cancer, breast cancer, gastric cancer, pancreatic cancer, head and neck cancer, colorectal cancer, kidney cancer, cervical cancer, ovarian cancer, endometrial cancer, uterine cancer, melanoma, pharyngeal cancer, oral cancer, skin cancer, lung cancer, glioblastoma multiforme, glioblastoma, urothelial carcinoma, prostate cancer, bladder cancer, gastrointestinal stromal tumor, squamous cell carcinoma, peritoneal cancer, liver cancer, salivary gland carcinoma, vulvar cancer, thyroid cancer, testicular cancer, anal cancer, or penile cancer.

*Linker-Drug Intermediate Compound*

In some aspects, the present disclosure provides a linker-drug intermediate compound having a structure of formula III below:

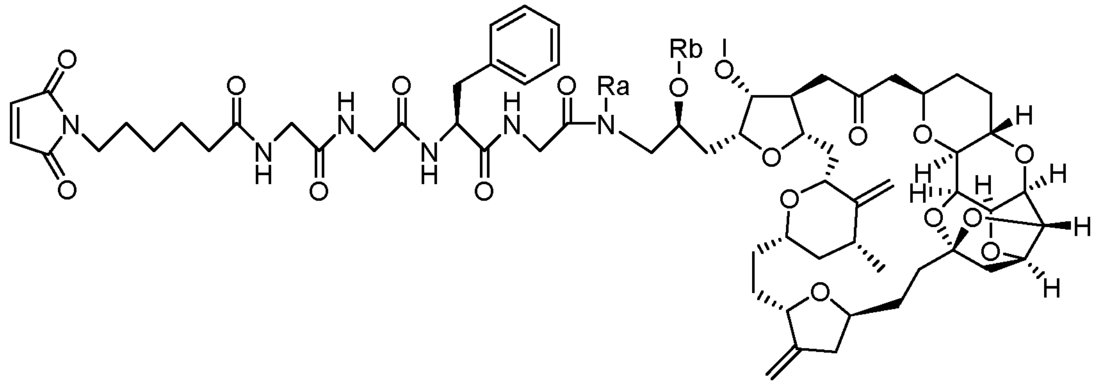

III,

wherein,

$R^a$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;

$R^b$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl; or

$R^a$ and $R^b$, together with an atom to which they are attached, form optionally substituted 5-8 membered heterocyclyl.

In some embodiments, in the linker-drug intermediate compound having the structure of formula III, $R^a$ and $R^b$ are each independently selected from a hydrogen atom, methyl, ethyl, propyl, and isopropyl.

In one specific embodiment, the present disclosure provides a linker-drug intermediate compound having a structure of formula III-1 below,

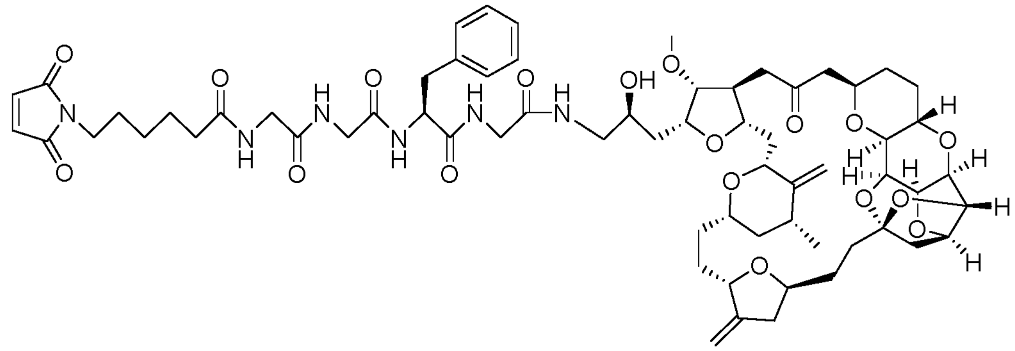

III-1.

The linker structure used in the present disclosure links the anti-tumor compound eribulin or a derivative thereof to an antibody or an antigen-binding fragment thereof, and the antibody-drug conjugate provided realizes excellent anti-tumor effect and/or safety. In some embodiments, the anti-tumor effect and/or safety of the antibody-drug conjugate is superior to eribulin. In some embodiments, the anti-tumor effect and/or safety of the antibody-drug conjugate is superior to that of the anti-HER3 antibody-drug conjugate U3-1402 (patritumab deruxtecan) of Daiichi Sankyo Co., Ltd. In some embodiments, the anti-HER3 antibody-drug conjugate provided exhibits good killing activity for tumor cells, for a variety of tumor cells, and/or for cells with different (high and/or medium and/or low) HER3 expression levels. In some embodiments, the anti-HER3 antibody-drug conjugate provided exhibits good killing activity against trastuzumab-resistant tumor cells. In some embodiments, the anti-HER3 antibody-drug conjugate provided has excellent safety. In some embodiments, the antibody-drug conjugate provided, such as the anti-HER3 antibody-drug conjugate, is less prone to aggregation. In some experiments, the anti-aggregation property of antibody-drug conjugates can be improved by using the linker structure disclosed herein to link the anti-tumor compound eribulin or a derivative thereof to an antibody or an antigen-binding fragment thereof.

## BRIEF DESCRIPTION OF THE DRAWINGS

FIGs. 1A-1G show the binding activities of patritumab-eribulin conjugates with different DAR values, patritumab-DDDXd-D8, and patritumab to cells with different HER3 expression levels;

FIGs. 2A-2D show the endocytosis of patritumab-eribulin conjugates with different DAR values, patritumab-DDDXd-D8, and patritumab in cells with different HER3 expression levels;

FIGs. 3A-3I show the cell killing rates of patritumab-eribulin conjugates with different DAR values and patritumab-DDDXd-D8 for cells with different HER3 expression levels;

FIG. 4 shows the effect of patritumab-eribulin conjugates with different DAR values, patritumab-DDDXd-D8, and vehicle control on the change in tumor volume of mice in nude mouse subcutaneous xenograft tumor models of JIMT-1 human breast cancer cells;

FIG. 5 shows the effect of patritumab-eribulin conjugates with different DAR values, patritumab-DDDXd-D8, and vehicle control on the tumor weight of mice in nude mouse subcutaneous xenograft tumor models of JIMT-1 human breast cancer cells; and

FIG. 6 shows the effect of patritumab-eribulin conjugates with different DAR values, patritumab-DDDXd-D8, and vehicle control on the weight change of mice in nude mouse subcutaneous xenograft tumor models of JIMT-1 human breast cancer cells.

## Explanation and definitions

Unless otherwise stated, the following terms used in the present application shall have the following meanings. A certain term, unless otherwise specifically defined, should not be considered uncertain or unclear, but construed according to its common meaning in the field. When referring to a trade name, it is intended to refer to its corresponding commercial product or its active ingredient.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted and oxo is not possible on an aromatic group.

The term "optional" or "optionally" means that the subsequently described event or circumstance may, but does not necessarily, occur. The description includes instances where the event or circumstance occurs and instances where it does not. The term "optionally substituted" refers to substituted or unsubstituted. For example, ethyl being "optionally" substituted with halogen means that the ethyl may be unsubstituted ($CH_2CH_3$), monosubstituted (for example, $CH_2CH_2F$), polysubstituted (for example, $CHFCH_2F$ and $CH_2CHF_2$), or fully substituted ($CF_2CF_3$). It will be appreciated by those skilled in the art that for any groups comprising one or more substituents, any substitutions or substituting patterns that may not exist spatially or cannot be synthesized are not introduced.

$C_{m-n}$ as used herein means that the moiety has an integer or a decimal number of carbon atoms in the given range. For example, "$C_{1-6}$" means that the group may have 1 carbon atom, 2 carbon atoms, 3 carbon atoms, 4 carbon atoms, 5 carbon atoms, or 6 carbon atoms.

When any variable (e.g., R) occurs once or more in the constitution or structure of a compound, the definition of the variable in each case is independent. Therefore, for example, if a group is substituted with 2 R, the definition of each R is independent.

When the number of connecting groups is 0, for example, $-(CH_2)_0-$, it means that the connecting group is a covalent bond.

When a variable is selected from a covalent bond, it means that the two groups it connects are directly connected. For example, in A-L-Z, when L represents a covalent bond, it means that the structure is actually A-Z.

The term "halo-" or "halogen" refers to fluorine, chlorine, bromine, and iodine.

The term "hydroxy" refers to -OH group.

The term "cyano" refers to -CN group.

The term "sulfydryl" refers to -SH group.

The term "amino" refers to $-NH_2$ group.

The term "nitro" refers to $-NO_2$ group.

The term "alkyl" refers to hydrocarbyl with a general formula of $C_nH_{2n+1}$. The alkyl may be linear or branched. For example, the term "$C_{1-6}$ alkyl" refers to alkyl containing 1 to 6 carbon atoms (for example, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like). The alkyl moieties (i.e., alkyl) of alkoxy, alkylamino, dialkylamino, alkylsulfonyl, and alkylthio are similarly defined as above.

The term "alkoxyl" refers to -O-alkyl.

The term "alkylamino" refers to -NH-alkyl.

The term "dialkylamino" refers to $-N(alkyl)_2$.

The term "alkylsulfonyl" refers to $-SO_2$-alkyl.

The term "alkylthio" refers to -S-alkyl.

The term "alkenyl" refers to linear or branched unsaturated aliphatic hydrocarbyl consisting of carbon atoms and hydrogen atoms with at least one double bond. Non-limiting examples of alkenyl include, but are not limited to, ethenyl, 1-propenyl, 2-propenyl, 1-butenyl, isobutenyl, 1,3-butadienyl, and the like.

The term "alkynyl" refers to linear or branched unsaturated aliphatic hydrocarbyl consisting of carbon atoms and hydrogen atoms with at least one triple bond. Non-limiting examples of alkynyl include, but are not limited to, ethynyl (-C≡CH), 1-propinyl ($-C≡C-CH_3$), 2-propinyl ($-CH_2-C≡CH$), 1,3-butadiynyl (-C≡C-C≡CH), and the like. The term "cycloalkyl" refers to a carbon ring that is fully saturated and may exist in the form of a monocyclic, bridged cyclic, or spiro cyclic structure. Unless otherwise specified, the carbon ring is usually a 3-10-membered ring. Non-limiting examples of cycloalkyl include, but are not limited to, cyclopropyl, cyclobutyl, cyclopentyl, cyclohexyl, norbomyl(bicyclo[2.2.1]heptyl), bicyclo[2.2.2]octyl, adamantyl, and the like.

The term "cycloalkenyl" refers to a non-aromatic carbon ring that is not fully saturated and may exist in the form of a monocyclic, bridged cyclic, or spiro cyclic structure. Unless otherwise specified, the carbon ring is usually a 5-8 membered ring. Non-limiting examples of cycloalkenyl include, but are not limited to, cyclopentenyl, cyclopentadienyl, cyclohexenyl, cyclohexadienyl, cycloheptenyl, cycloheptadienyl, and the like.

The term "heterocyclyl" refers to a fully saturated or partially unsaturated (but not fully unsaturated heteroaromatic group) nonaromatic ring that may exist in the form of a monocyclic, bridged cyclic, or spiro cyclic structure. Unless otherwise specified, the heterocyclyl is usually a 3-7 membered ring containing 1-3 heteroatoms (preferably 1 or 2 heteroatoms) independently selected from sulfur, oxygen, and/or nitrogen. Non-limiting examples of heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4*H*-pyranyl, morpholinyl, sulfomorpholinyl, tetrahydrothienyl, and the like.

The term "heterocycloalkyl" refers to a fully saturated cyclic group that may exist in the form of a monocyclic, bridged cyclic, or spiro cyclic structure. Unless otherwise specified, the heterocyclyl is usually a 3-7 membered ring containing 1-3 heteroatoms (preferably 1 or 2 heteroatoms) independently selected from sulfur, oxygen, and/or nitrogen. Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azacycloheptanyl, oxacycloheptanyl, and thiocycloheptanyl. Monocyclic heterocycloalkyl having 5 or 6 ring atoms is preferred.

The term "aryl" refers to an all-carbon aromatic monocyclic or fused polycyclic group having a conjugated $\pi$-electron system. For example, aryl may have 6-20 carbon atoms, 6-14 carbon atoms, or 6-12 carbon atoms. Non-limiting examples of aryl include, but are not limited to, phenyl, naphthyl, anthryl, 1,2,3,4-tetrahydronaphthalene, and the like.

The term "heteroaryl" refers to a monocyclic or fused polycyclic system that comprises at least one ring atom selected from N, O, and S, with the remaining ring atoms being C, and that has at least one aromatic ring. Preferably, heteroaryl has a single 4-8 membered ring, in particular a 5-8 membered ring, or has a plurality of fused rings comprising 6-14 ring atoms, in particular 6-10 ring atoms. Non-limiting examples of heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

The "derivative": a compound formed by substituting atoms or atom groups in the molecule of the parent compound with other atoms or atom groups is referred to as a derivative of the parent compound.

The compounds and intermediates disclosed herein may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerism and imine-enamine isomerization. A specific example of a proton tautomer is an imidazole moiety where a proton can transfer between two ring nitrogens. A valence tautomer includes the interconversion via recombination of some bonding electrons.

The compound disclosed herein can be asymmetrical, for example, having one or more stereoisomers. Unless otherwise stated, all stereoisomers are included, for example, enantiomers and diastereoisomers. The compound with asymmetrical carbon atoms disclosed herein can be separated in an optically pure form or in a racemic form. The optically pure form can be separated from a racemic mixture or can be synthesized using a chiral raw material or a chiral reagent.

Any atom of a compound labeling-synthesized herein may represent any stable isotope of the atom, if not specifically designated. Unless otherwise specified, when a position in a structure is defined as H, i.e., hydrogen (H-1),

this position contains only the naturally occurring isotope. Similarly, unless otherwise specified, when a position in a structure is defined as D, i.e., deuterium (H-2), this position contains an isotope having an amount that is at least 3340 times greater than the amount of the naturally occurring isotope (0.015%) (i.e., at least 50.1% deuterium isotope); when one or more positions in the structure of the compound labeling-synthesized are defined as D, i.e., deuterium (H-2), the content of the compound represented by the structure may be at least 52.5%, at least 60%, at least 67.5%, at least 75%, at least 82.5%, at least 90%, at least 95%, at least 97%, at least 98.5%, at least 99%, or at least 99.5%. The deuterated ratio of a compound labeling-synthesized herein refers to a ratio of the content of the labeled synthetic isotope to the content of the naturally occurring isotope. The deuterated ratio per designated deuterium atom of the compound labeling-synthesized herein may be at least 3500 times (52.5%), at least 4000 times (60%), at least 4500 times (67.5%), at least 5000 times (75%), at least 5500 times (82.5%), at least 6000 times (90%), at least 6333.3 times (95%), at least 6466.7 times (97%), at least 6566.7 times (98.5%), at least 6600 times (99%), or at least 6633.3 times (99.5%). Isotopologues herein refer to compounds that differ only in isotopic composition in terms of chemical structure. The compound labeling-synthesized herein has the same chemical structure, with only isotopic changes in the atomic composition of its molecules. Therefore, the compound labeling-synthesized herein having deuterium at a specific position also contains very few hydrogen isotopologues at this position, and the amount of hydrogen isotopologue at a certain position in the compound labeling-synthesized herein depends on many factors, including the deuterium isotopic purity of the deuterated agent ($D_2O$, $D_2$, $NaBD_4$, $LiAlD_4$, and the like) and the effectiveness of the synthesis methods for introducing deuterium isotope. However, as previously mentioned, the total amount of such hydrogen isotopologue at a certain position will be less than 49.9%. The total amount of hydrogen isotopologue at a certain position in the compound labeling-synthesized herein will be less than 47.5%, 40%, 32.5%, 25%, 17.5%, 10%, 5%, 3%, 1%, or 0.5%.

In the present disclosure, any individual atom not designated as deuterium is present at its natural isotopic abundance.

As used herein, the term "bystander effect" refers to an effect in which a cytotoxic drug conjugated to an antibody or an antigen-binding fragment thereof via a cleavable or non-cleavable linker has the ability to diffuse and penetrate through the cell membrane after release from the antibody or the antigen-binding fragment thereof and thereby result in killing of adjacent cells. The ability to diffuse and penetrate through the cell membrane is related to the hydrophobicity of the cytotoxic drug or the combination of the cytotoxic drug and the linker. Such cytotoxic drugs may be, e.g., eribulin or MMAE. The bystander effect may be desirable particularly in tumors with heterogeneous target expression and solid tumors where antibody penetration may be limited.

The term "treating" refers to administering the compound or pharmaceutical composition described herein to prevent, ameliorate, or eliminate a disease or one or more symptoms associated with the disease, and includes, but is not limited to:

(i) preventing the occurrence of a disease or disease state in a mammal, particularly when such a mammal is predisposed to the disease state but has not yet been diagnosed as having it;
(ii) inhibiting a disease or disease state, i.e., arresting its progression;
(iii) alleviating a disease or disease state, i.e., causing its regression; and
(iv) reducing any direct or indirect pathological consequences of a disease or disease state.

The term "therapeutically effective amount" refers to an amount of the compound disclosed herein for (i) treating or preventing a specific disease, condition, or disorder; (ii) alleviating, ameliorating, or eliminating one or more symptoms of a specific disease, condition or disorder, or (iii) preventing or delaying onset of one or more symptoms of a specific disease, condition, or disorder described herein. The amount of the compound or the pharmaceutical composition disclosed herein that constitutes a "therapeutically effective amount" may vary depending on factors such as the compound or the pharmaceutical composition and its ability to elicit a desired response in an individual, the disease state and its severity, the mode of administration, and the age, sex, and weight of the mammal to be treated. The effective amount may also be determined routinely by those skilled in the art in accordance with their knowledge and the content of the present disclosure.

The term "pharmaceutically acceptable" is used for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "solvate" refers to a substance formed by association of a compound with a solvent molecule.

The term "antibody" is used in its broadest sense and specifically encompasses intact monoclonal antibodies, polyclonal antibodies, multispecific antibodies (e.g., bispecific antibodies) formed from at least two intact antibodies,

multifunctional antibodies, and antibody fragments, so long as they possess the desired biological activity.

The term "humanized antibody" refers to an antibody comprising CDRs derived from a non-human antibody, and the remainder of the antibody molecule is derived from one or more human antibodies.

The term "mutant" is used to refer to a peptide comprising an amino acid sequence derived from the amino acid sequence of the peptide as follows: substitution of one or two or more amino acids with amino acids different from the original peptide, deletion of one or two or more wild-type amino acids, insertion of one or two or more amino acids that do not exist in the wild type, and/or addition of amino acids that do not exist in the wild type to the amino terminus (N-terminus) and/or the carboxy terminus (C-terminus) of the wild type (collectively referred to as "mutation"). In the present disclosure, "insertion" may also be included in "addition".

The term "CDR" (complementarity determining region), also known as "hypervariable region", refers to each region of an antibody variable domain which is highly variable in sequence and/or forms a structurally defined loop. Natural four-chain antibodies typically comprise six CDRs, three in the heavy chain variable region and three in the light chain variable region.

The term "variable region" refers to a domain of about 100 to 110 or more amino acids defined by the N-terminal domain of the light or heavy chain of an antibody and primarily responsible for antigen recognition. The terms light chain variable region (VL) and heavy chain variable region (VH) refer to these light chain and heavy chain domains, respectively.

The term "Fab" means comprising the constant domain (CL) of the light chain and the first constant domain (CH1) of the heavy chain, together with the variable domains VL (light chain variable region) and VH (heavy chain variable region) in the light chain and heavy chain, respectively. The variable domain comprises complementarity determining regions (CDRs) that are involved in antigen binding.

The term "scFv" comprises the VH and VL domains of an antibody, wherein these domains are present in a single polypeptide chain. In some embodiments, scFv further comprises a polypeptide linker between the VH and VL domains that enables the scFv to form the required structure for antigen binding.

The term "antibody moiety" refers to the antibody moiety in an antibody-drug conjugate. In certain specific embodiments, the antibody moiety is linked to an intermediate linker moiety via a specific functional group, and the antibody moiety can specifically bind to an antigen.

The term "linker moiety" refers to a part of the antibody-drug conjugate that links an antibody moiety to a cytotoxic drug moiety and it may be cleavable or uncleavable; the cleavable linker may be cleaved in a target cell to release the cytotoxic drug.

The term "cytotoxic drug moiety" refers to a cytotoxic drug moiety in an antibody-drug conjugate. In certain specific embodiments, the cytotoxic drug moiety is linked to the intermediate linker moiety via a functional group, so that cytotoxic drug molecules can be liberated in tumor cells to exert an anti-tumor effect.

The term "trastuzumab" (generic name) refers to a recombinant humanized monoclonal antibody that selectively acts on ECD4 of human epidermal growth factor receptor-2 (HER2) and can be used to treat HER2-positive cancer, an example of which is the commercially available therapeutic monoclonal antibody product under the trade name HERCEPTIN®.

The term "patritumab" is a fully human anti-HER3 monoclonal antibody and can be used to treat cancer expressing the HER3 protein.

The term "HER2" is the second member of the EGFR family and has a tyrosine kinase activity, wherein HER2 expression levels can be detected by immunohistochemical assay; HER2-positive refers to IHC3+, HER2-negative refers to IHC1+/0, and for IHC2+, ISH assay should be performed for further confirmation.

The term "HER3" (human epidermal growth factor receptor 3, also known as ErbB3) is a receptor protein tyrosine kinase and belongs to the epidermal growth factor receptor (EGFR) subfamily of receptor protein tyrosine kinases, which also includes HER1 (also known as EGFR), HER2, and HER4. HER3 is a transmembrane receptor and is composed of an extracellular ligand binding domain (ECD), a dimerization domain within the ECD, a transmembrane domain, an intracellular protein tyrosine kinase domain (TKD), and a C-terminal phosphorylation domain. HER3 has been found to be overexpressed in several types of cancers (e.g., breast cancer, gastrointestinal cancer, and pancreatic cancer). A correlation between expression of HER2/HER3 and progression from non-invasive to invasive stages has been shown.

The term "triple-negative breast cancer" is breast cancer that is negative for expression of estrogen receptors, progesterone receptors, and human epidermal growth factor receptor-2.

The term "$EC_{50}$" refers to the effective concentration that induces 50% of the maximal response of an antigen-binding construct. $EC_{50}$ can be measured by ELISA or FACS analysis or any other method known in the art.

The term "identity" is also known as consistency. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. Alignment of amino acid sequences for identity

can be performed in a variety of ways within the skill in the art, e.g., BLAST, BLAST-2, ALIGN, or Megalign (DNASTAR) software. Those skilled in the art can determine suitable parameters for aligning sequences, including any algorithms required to obtain maximum alignment for the full length of sequences being compared.

The terms "subject", "patient" and "subject" are used interchangeably herein. In some embodiments, the term "subject", "patient", or "subject" is a mammal. In some embodiments, the subject, patient, or subject is a mouse. In some embodiments, the subject, patient, or subject is a human being.

As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, "about" may mean a range of up to ± 5%, for example, fluctuating within a particular numerical range given ± 2%, ± 1% or ± 0.5%. Where a particular value is given in the present application or in the patent scope of disclosure application, unless otherwise stated, "about" should be considered to mean being within an acceptable error range for that particular value. Herein, unless otherwise stated, the values of the parameters or conditions in a step are all modified by "about" by default.

## DETAILED DESCRIPTION

The present disclosure further provides the following specific embodiments, but the scope of the present disclosure is not limited thereto:

Embodiment 1. An antibody-drug conjugate having general formula Ab-$(L-U)_n$ or a pharmaceutically acceptable salt or a solvate thereof, wherein Ab represents an antibody moiety, L represents a linker moiety, U represents a cytotoxic drug moiety, and n is an integer or a decimal selected from numbers from 1 to 10, wherein the antibody-drug conjugate comprises a structure of formula IIa below:

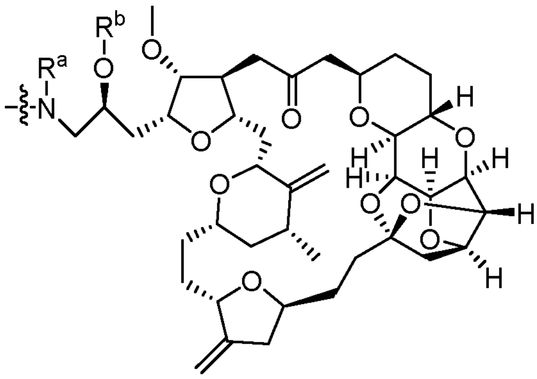

IIa,

wherein,

$R^a$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;

$R^b$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;

or,

$R^a$ and $R^b$, together with an atom to which they are attached, form optionally substituted 5-8 membered heterocyclyl.

Embodiment 2. The antibody-drug conjugate or the pharmaceutically acceptable salt or the solvate thereof according to Embodiment 1, wherein the antibody-drug conjugate comprises a structure of formula IIIa below:

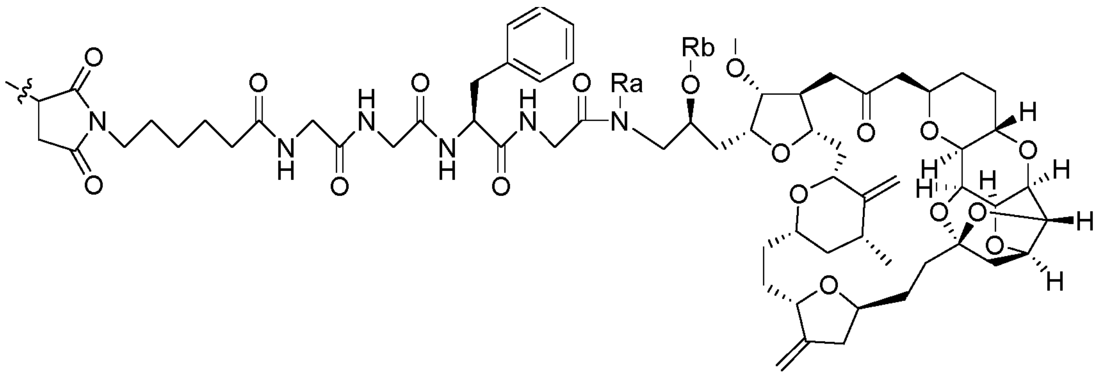

IIIa.

Embodiment 3. The antibody-drug conjugate or the pharmaceutically acceptable salt or the solvate thereof according to Embodiment 1 or 2, wherein the antibody-drug conjugate is a structure of formula IV below:

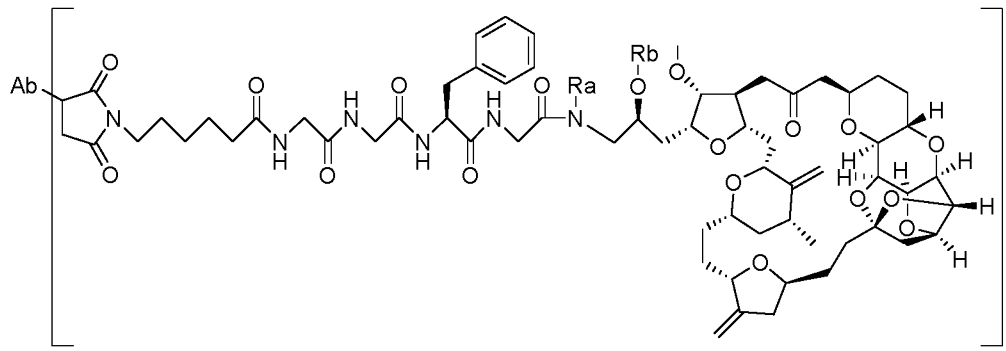

IV,

wherein,

Ab represents an antibody moiety, and
n is an integer or a decimal selected from numbers from 1 to 10.

Embodiment 4. The antibody-drug conjugate or the pharmaceutically acceptable salt or the solvate thereof according to any one of Embodiments 1-3, wherein $R^a$ and $R^b$ are each independently selected from a hydrogen atom, methyl, ethyl, propyl, and isopropyl.

Embodiment 5. The antibody-drug conjugate or the pharmaceutically acceptable salt or the solvate thereof according to Embodiment 1 or 2, wherein the antibody-drug conjugate comprises a structure of formula IIIa-1 below:

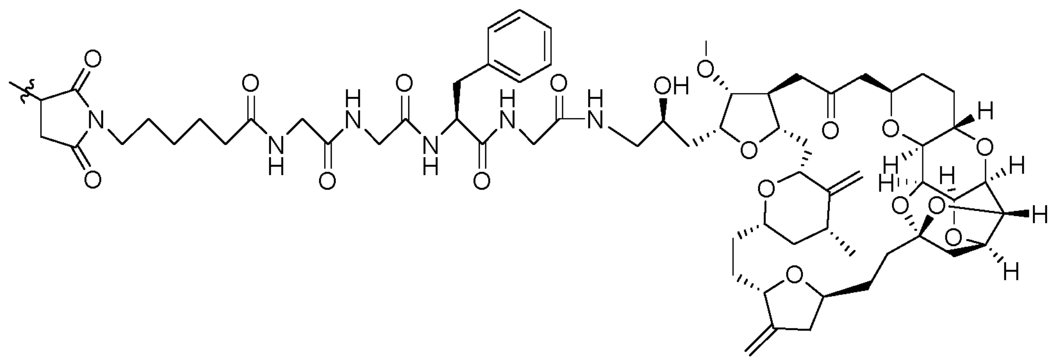

IIIa-1.

Embodiment 6. The antibody-drug conjugate or the pharmaceutically acceptable salt or the solvate thereof according to Embodiment 3, wherein the antibody-drug conjugate is a structure of formula IV-1 below:

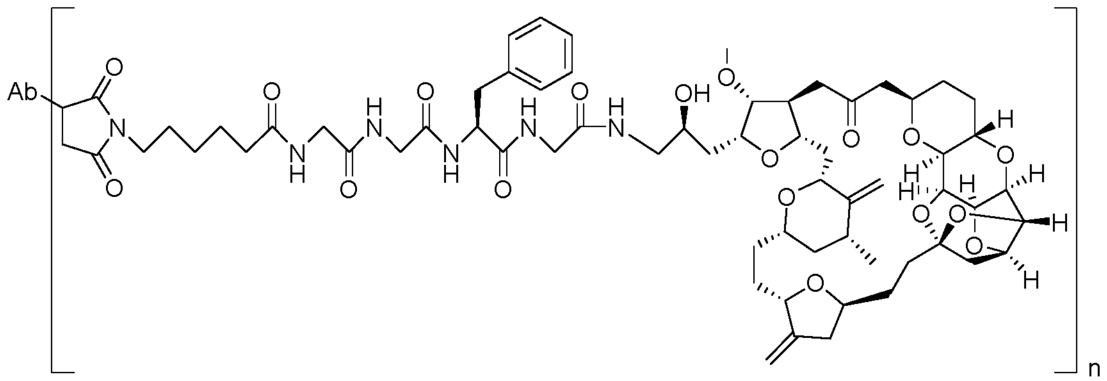

IV-1.

Embodiment 7. The antibody-drug conjugate or the pharmaceutically acceptable salt or the solvate thereof according to any one of Embodiments 1-6, wherein n is 2-4.8, 2.6-4.8, 3.5-4.8, 4-4.8, 2-4.5, 2.6-4.5, 3.5-4.5, 4-4.5, 3.5-4.2, 3.5-4, 4-4.2, 7-8, 7-7.9, 7-7.6, 7-7.5, 7.1-8, 7.1-7.9, 7.1-7.6, 7.5-8, 7.6-8, or 7.6-7.9.

Embodiment 8. The antibody-drug conjugate or the pharmaceutically acceptable salt or the solvate thereof according to Embodiment 7, wherein n is about 2.6, about 4, about 4.2, about 4.8, about 7, about 7.1, about 7.5, about 7.6, about 7.9, or about 8.

Embodiment 9. The antibody-drug conjugate or the pharmaceutically acceptable salt or the solvate thereof according to any one of Embodiments 1-8, wherein Ab is an anti-HER3 antibody or an antigen-binding fragment thereof.

Embodiment 10. The antibody-drug conjugate or the pharmaceutically acceptable salt or the solvate thereof according to Embodiment 9, wherein the anti-HER3 antibody or the antigen-binding fragment thereof comprises: HCDR1 comprising an amino acid sequence set forth in SEQ ID NO: 1, HCDR2 comprising an amino acid sequence set forth in SEQ ID NO: 2, HCDR3 comprising an amino acid sequence set forth in SEQ ID NO: 3, LCDR1 comprising an amino acid sequence set forth in SEQ ID NO: 4, LCDR2 comprising an amino acid sequence set forth in SEQ ID NO: 5, and LCDR3 comprising an amino acid sequence set forth in SEQ ID NO: 6.

Embodiment 11. The antibody-drug conjugate or the pharmaceutically acceptable salt or the solvate thereof according to Embodiment 10, wherein the anti-HER3 antibody or the antigen-binding fragment thereof comprises a heavy chain variable region and a light chain variable region, wherein the heavy chain variable region comprises an amino acid sequence having at least 80% identity to an amino acid sequence set forth in SEQ ID NO: 7 and the light chain variable region comprises an amino acid sequence having at least 80% identity to an amino acid sequence set forth in SEQ ID NO: 8, or the heavy chain variable region comprises the amino acid sequence set forth in SEQ ID NO: 7 and the light chain variable region comprises the amino acid sequence set forth in SEQ ID NO: 8.

Embodiment 12. The antibody-drug conjugate or the pharmaceutically acceptable salt or the solvate thereof according to Embodiment 10 or 11, wherein the anti-HER3 antibody or the antigen-binding fragment thereof comprises a heavy chain and a light chain, wherein the heavy chain comprises an amino acid sequence having at least 80% identity to an amino acid sequence set forth in SEQ ID NO: 9, and the light chain comprises an amino acid sequence having at least 80% identity to an amino acid sequence set forth in SEQ ID NO: 10.

Embodiment 13. The antibody-drug conjugate or the pharmaceutically acceptable salt or the solvate thereof according to Embodiment 9, wherein the anti-HER3 antibody is patritumab.

Embodiment 14. The antibody-drug conjugate or the pharmaceutically acceptable salt or the solvate thereof according to any one of Embodiments 9-13, wherein the antibody-drug conjugate or the pharmaceutically acceptable salt or the solvate thereof exhibits one of or a combination of several of the following properties:

(a) binding to HER3;
(b) blocking binding of HER3 to a ligand;
(c) showing endocytosis in cells expressing HER3;
(d) having killing activity against HER3-expressing tumor cells; and
(e) having bystander effect.

Embodiment 15. A pharmaceutical composition, comprising the antibody-drug conjugate or the pharmaceutically acceptable salt or the solvate thereof according to any one of Embodiments 1-14, wherein optionally, the pharmaceutical composition further comprises a pharmaceutically acceptable carrier.

Embodiment 16. Use of the antibody-drug conjugate or the pharmaceutically acceptable salt or the solvate thereof according to any one of Embodiments 1-14 or the pharmaceutical composition according to Embodiment 15 for preparing a medicament for treating cancer, wherein preferably, the cancer is HER3-positive cancer; preferably,

the cancer is biliary tract cancer, carcinosarcoma, esophageal cancer, esophagogastric junction cancer, breast cancer, gastric cancer, pancreatic cancer, head and neck cancer, colorectal cancer, kidney cancer, cervical cancer, ovarian cancer, endometrial cancer, uterine cancer, melanoma, pharyngeal cancer, oral cancer, skin cancer, lung cancer, glioblastoma multiforme, glioblastoma, urothelial carcinoma, prostate cancer, bladder cancer, gastrointestinal stromal tumor, squamous cell carcinoma, peritoneal cancer, liver cancer, salivary gland carcinoma, vulvar cancer, thyroid cancer, testicular cancer, anal cancer, or penile cancer.

Embodiment 17. A method for treating cancer, comprising administering to a patient in need thereof a therapeutically effective amount of the antibody-drug conjugate or the pharmaceutically acceptable salt or the solvate thereof according to any one of Embodiments 1-14 or the pharmaceutical composition according to Embodiment 15, wherein preferably, the cancer is HER3-positive cancer.

Embodiment 18. The method according to Embodiment 17, comprising contacting tumor cells with the antibody-drug conjugate or the pharmaceutically acceptable salt or the solvate thereof or the pharmaceutical composition, thereby killing the tumor cells or inhibiting growth of the tumor cells.

Embodiment 19. The method according to Embodiment 17 or 18, wherein the cancer is biliary tract cancer, carcinosarcoma, esophageal cancer, esophagogastric junction cancer, breast cancer, gastric cancer, pancreatic cancer, head and neck cancer, colorectal cancer, kidney cancer, cervical cancer, ovarian cancer, endometrial cancer, uterine cancer, melanoma, pharyngeal cancer, oral cancer, skin cancer, lung cancer, glioblastoma multiforme, glioblastoma, urothelial carcinoma, prostate cancer, bladder cancer, gastrointestinal stromal tumor, squamous cell carcinoma, peritoneal cancer, liver cancer, salivary gland carcinoma, vulvar cancer, thyroid cancer, testicular cancer, anal cancer, or penile cancer.

Embodiment 20. A linker-drug intermediate compound having a structure of formula III:

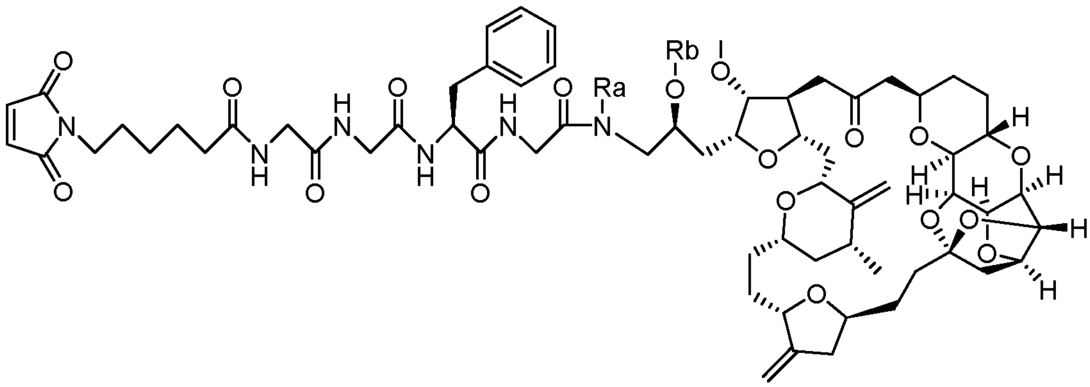

III,

wherein,

$R^a$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;

$R^b$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl; or

$R^a$ and $R^b$, together with an atom to which they are attached, form optionally substituted 5-8 membered heterocyclyl.

Embodiment 21. The linker-drug intermediate compound according to Embodiment 20, wherein $R^a$ and $R^b$ are each independently selected from a hydrogen atom, methyl, ethyl, propyl, and isopropyl.

For clarity, the present disclosure is further described with the following examples, which are, however, not intended to limit the scope of the present application. The reagents used herein are commercially available and can be used without further purification.

Patritumab used in the examples of the present application was prepared according to a conventional method for antibody, that is, an expression vector (including, for example, pcDNA3.1 vector disclosed in CN107001463A, pCHO1.0 vector disclosed in CN109422811A, etc.) was constructed and transfected into Expi-CHO host cells for expression, followed by purification with Protein A affinity chromatography; the amino acid sequences of the heavy and light chains of patritumab are set forth in SEQ ID NOs: 9 and 10, respectively. For synthesis of deuterated MC-GGFG-DXd (MC-GGFG-DDDXd), reference is made to the method in Example 14 of patent publication WO2022033578A1.

The cells involved in the examples of the present application and their sources are shown in the table below:

| Cells | Source |
|---|---|
| NCI-N87 | Cell Bank of the Chinese Academy of Sciences |
| BT474 | Chinese Academy of Sciences |
| SKBR3 | Crown Bioscience |
| SK-OV3 | Cell Bank of the Chinese Academy of Sciences |
| JIMT-1 | ATCC |
| Capan1 | Beina Bio |
| MCF-7 | Cell Bank of the Chinese Academy of Sciences |
| KYSE410 | Shanghai Yaji Biotechnology Co., Ltd. |
| MDA-MB-468 | Beina Bio |
| HCC1569 | Nanjing Cobioer |
| SW620 | Nanjing Cobioer |
| A549 | Cell Culture Center, Institute of Basic Medical Sciences, Chinese Academy of Medical Sciences |
| WiDr | Nanjing Cobioer |

## Example 1: Preparation of Compound III-1

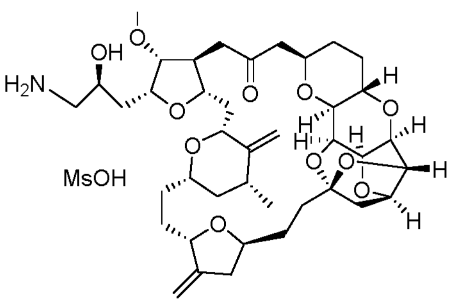

A

+

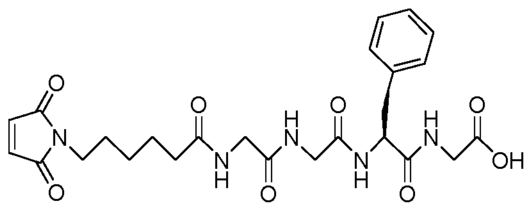

B

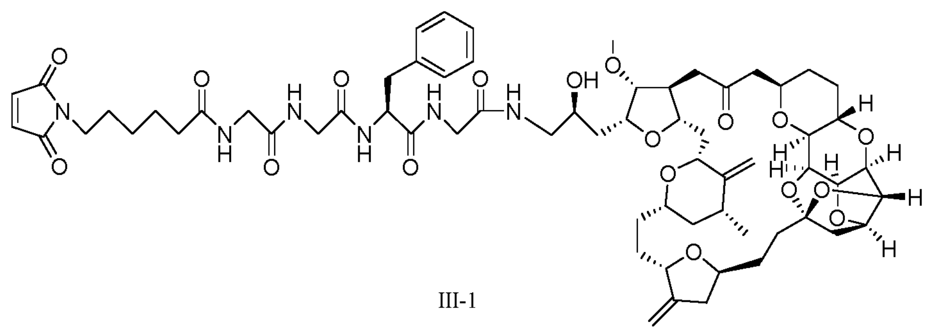

III-1

100 mg of compound A (0.12 mmol) and 75 mg of compound B (0.145 mmol) were weighed out and added into a 20 mL reaction tube (CAS No. of compound A: 2413428-36-9; CAS No. of compound B: 441045-17-6), and 2 mL of *N,N*-dimethylformamide was added. The mixture was cooled to 0 °C, followed by addition of 70 mg of 2-(7-azabenzotriazole)-*N*,*N*,*N'*,*N'*-tetramethyluronium hexafluorophosphate (0.18 mmol) and 49 mg of *N*,*N*-diisopropylethylamine (0.36 mmol). The mixture was reacted for 1 h at 0 °C and purified by preparative liquid chromatography to obtain about

70 mg of compound III-1 (MC-GGFG-eribulin). Compound III-1 has M/z of 1241.72[M+H]$^+$, as analyzed by ESI-MS. The hydrogen spectrum of compound III-1 is as follows:
1H NMR (500 MHz, DMSO) δ 8.22 (t, J = 5.3 Hz, 1H), 8.12 (d, J = 8.0 Hz, 1H), 8.06 (t, J = 5.4 Hz, 1H), 7.99 (t, J = 5.3 Hz, 1H), 7.65 (d, J = 5.2 Hz, 1H), 7.30-7.21 (m, 4H), 7.18 (d, J = 6.4 Hz, 1H), 6.99 (s, 2H), 5.02 (d, J = 26.0 Hz, 2H), 4.79 (d, J = 38.9 Hz, 2H), 4.65-4.60 (m, 2H), 4.56 (d, J = 3.7 Hz, 1H), 4.50-4.42 (m, 1H), 4.26 (d, J = 10.1 Hz, 1H), 4.21-4.14 (m, 1H), 4.10 (s, 3H), 4.05-3.98 (m, 1H), 3.86-3.64 (m, 8H), 3.60-3.45 (m, 4H), 3.37-3.30 (m, 2H), 3.26 (s, 4H), 3.17-3.09 (m, 1H), 3.08-2.99 (m, 2H), 2.84 (d, J = 10.4 Hz, 1H), 2.86-2.66 (m, 3H), 2.56-2.50m, 1H), 2.37-2.18 (m, 5H), 2.15-2.05 (m, 3H), 2.05-1.96 (m, 2H), 1.95-1.84 (m, 4H), 1.75-1.57 (m, 6H), 1.55-1.38 (m, 6H), 1.35-1.25 (m, 3H), 1.23-1.12 (m, 3H), 1.03 (d, J = 6.0 Hz, 3H), 1.00-0.92 (m, 1H).

**Example 2: Preparation of Antibody-Drug Conjugates**

**Reagents:**

Solution A: pH 7.4 PBS buffer
Solution B: 10 mM aqueous TCEP (tris(2-carboxyethyl)phosphine hydrochloride)
Solution C: DMSO
Solution D: histidine buffer (containing 0.89 mg/mL L-histidine and 4.04 mg/mL L-histidine hydrochloride monohydrate)
Solution E: 700 mg/mL sucrose solution (prepared with solution D)
Solution F: 20 mg/mL Tween 80 (prepared with solution D)

Experimental procedures:

1. Buffer exchange of antibody

a. a 30 KD ultrafiltration centrifuge tube was fully wet using solution A;
b. the antibody was buffer-exchanged into solution A;
c. an appropriate amount of solution A was added to adjust the antibody concentration.

2. Antibody reduction

a. the molar weight of the antibody was calculated and recorded as N1;
b. an appropriate amount of solution B was added into the antibody solution to ensure that the molar weight of TCEP in the reaction system was N2;
c. the ultrafiltration centrifuge tube was wrapped with aluminum foil, placed on a rotary culture instrument, shaken at low speed (20 rpm), and reacted for 1 h at 37 °C in the dark.

3. Conjugation

a. an appropriate amount of linker-payload was dissolved in DMSO to adjust a final concentration to 10 mg/mL;
b. DMSO was added into the antibody solution to make the antibody concentration be 5%, and then an appropriate amount of linker-payload solution was added to make the molar weight be N3;
c. the ultrafiltration centrifuge tube was wrapped with aluminum foil, placed on a rotary culture instrument, shaken at low speed (20 rpm), and reacted for 1.5 h at 22 °C in the dark.

4. Termination of conjugation

a. an ultrafiltration centrifuge tube was wet using solution D;
b. the antibody was buffer-exchanged into solution D, an appropriate amount of solution E and solution F was added, and the mixture was cryopreserved at -80 °C.

**Determination of DAR values (average number of drugs linked per antibody molecule) of antibody-drug conjugates**

DAR values were determined by LC-MS method. 50 μg of the ADC sample was to 1 μL of glycosidase PNGase F (RHINO BIO, China) and the mixture was incubated at 37 °C for 20 h. The mass spectrometer used in the experiment was a high resolution Xevo G2-XS (Waters, USA). The concentration of the sample was adjusted to 5 μM, and mass spectrum data were collected in a positive ion mode by adopting a direct sampling method. The collected non-denaturing mass spectral data were analyzed and processed using the software UNIFI 1.8.2.169 (Waters, USA).

**Determination of protein concentration of antibody-drug conjugates**

Protein concentration was detected by lowry method. The absorbance values of samples at $OD_{650}$ wavelength were detected by using a microplate reader, a standard curve was fitted, the absorbance values of the samples were each substituted into the standard curve, and the protein concentration was calculated.

Antibody-drug conjugate of formula IV-1 (including IV-1 (patritumab)) and other antibody-drug conjugates of the formula IV series were prepared according to the above method:

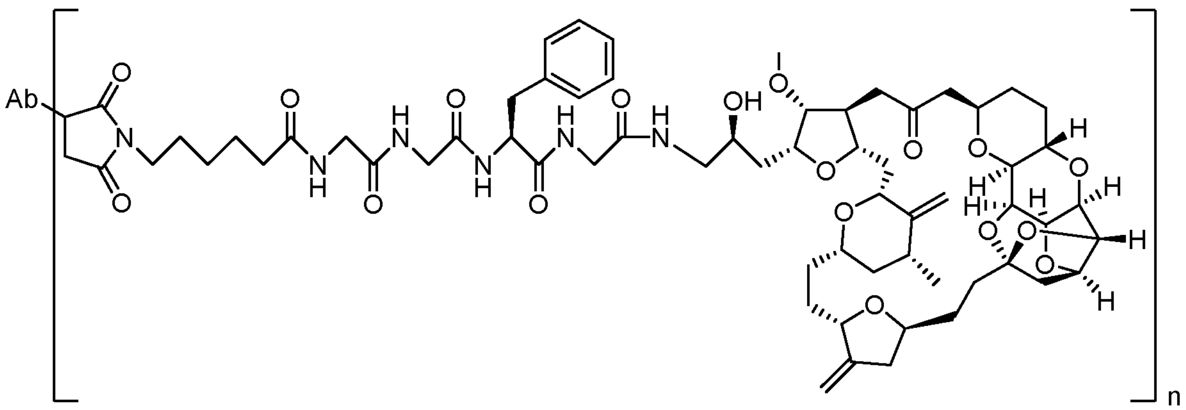

IV-1

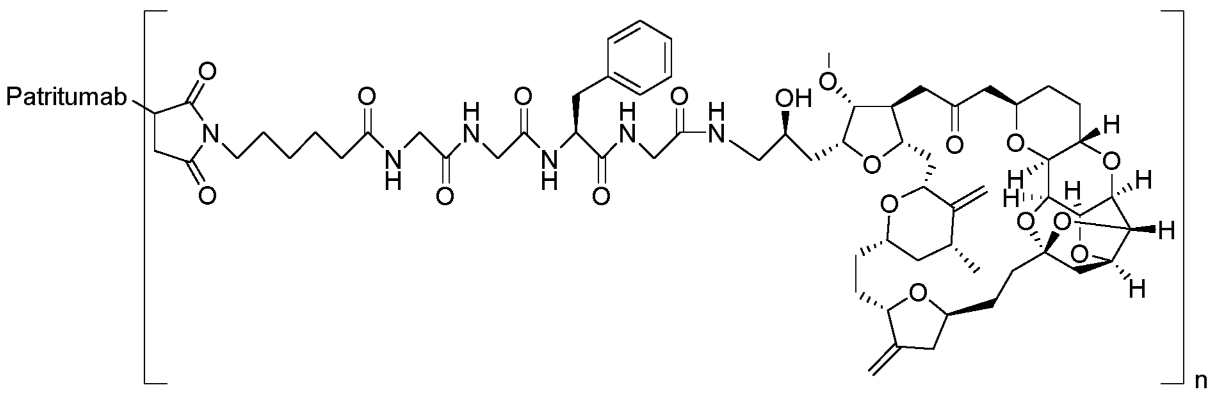

IV-1 (patritumab)

Sample 1 (DAR = 4.8, protein concentration = 1.71 mg/mL) and sample 2 (DAR = 7.1, protein concentration = 1.65 mg/mL) were prepared separately.

**Example 3: Cell Viability of Small Molecule Cytotoxic Compound and Antibody-Drug Conjugate**

A small molecule cytotoxic compound was diluted to 35,000 ng/mL-0.0896 ng/mL with medium, and 9 concentration points were obtained. Tumor cells in the logarithmic growth phase were collected, adjusted to a density of 1 × $10^5$ cells/mL, and plated at 100 μL per well, and a blank well without any cells was set as a control. The above serially diluted samples were each added at 50 μL per well. The plate was incubated in an incubator at 37 °C with 5% COz for

5 days. The culture medium was discarded, and CCK-8 (Dojindo, Japan, Cat. No.: CK04) working solution was added at 100 μL per well. The plate was incubated for 4-5 h for color developing and placed in a microplate reader (manufacturer: Thermo, Model: VarioskanFlash). The absorbance values at a wavelength of 450 nm were read and recorded using a reference wavelength of 630 nm. The proliferation inhibition rates against the tumor cells were calculated.

An antibody-drug conjugate was diluted to 5000 ng/mL-0.0128 ng/mL with medium, and 9 concentration points were obtained. Tumor cells in the logarithmic growth phase were collected, adjusted to a density of $2 \times 10^4$ cells/mL, and plated at 100 μL per well, and a blank well without any cells was set as a control. The above serially diluted samples were each added at 50 μL per well. The plate was incubated in an incubator at 37 °C with 5% COz. The culture medium was discarded, CTG detection medium (Promega, Cat. No.: G7572) was added at 100 μL per well, and the plate was incubated for 10 min for color developing and placed in a microplate reader (manufacturer: Thermo, model: Varioskan-Flash) to read the chemiluminescence value. The proliferation inhibition rates against the tumor cells were calculated.
cell activity of IV-1 (patritumab):

| Cell line | $EC_{50}$ (nM) | |
|---|---|---|
| | DAR=4.8 | DAR=7.1 |
| MCF-7 | 0.08 | 0.05 |
| BT474 | 0.07 | 0.04 |
| SKBR3 | 0.17 | 0.08 |

**Example 4: Preparation of Antibody-Drug Conjugates**

**Reagents:**

Solution G: histidine/histidine hydrochloride buffer (1.43 mg/mL L-histidine, 2.27 mg/mL L-histidine hydrochloride monohydrate);
Solution H: 10 mM aqueous TCEP (tris(2-carboxyethyl)phosphine hydrochloride);
Solution I: DMSO (dimethyl sulfoxide);
Solution J: 500 mg/mL sucrose solution (prepared with solution G);
Solution K: 30 mg/mL Tween 80 (prepared with solution G);
Solution L: solution G containing 10% DMSO;
Solution M: 0.3 M $Na_2HPO_4$;
Antibody: patritumab;

Linker-payload (linker-drug intermediate compound): MC-GGFG-eribulin, the compound having a structure of formula III-1 in Example 1 (used in the preparation of patritumab-eribulin conjugates); MC-GGFG-DDDXd (used in the preparation of patritumab-DDDXd conjugate).

(1) Patritumab-eribulin conjugates with a DAR of 2.6 or 7.6 were prepared separately according to the following experimental procedures and named patritumab-eribulin-D2 and patritumab-eribulin-D8, respectively; a patritumab-DDDXd conjugate with a DAR of 7.9 was prepared according to the following experimental procedures and named patritumab-DDDXd-D8:

**Experimental procedures:**

1. Buffer exchange of antibody:

A 30 KD ultrafiltration centrifuge tube was fully wet using solution G, the antibody was buffer-exchanged into solution G, and an appropriate amount of solution G was added to adjust the concentration of the antibody to 10 mg/mL; then, an appropriate amount of solution M was added to adjust the pH of the antibody solution to about 7.0.

2. Antibody reduction:

The molar weight of the antibody was calculated and recorded as N1; an appropriate amount of solution H was

added into the antibody solution to ensure that the molar weight of TCEP in the reaction system was N2; the resulting mixture was shaken at 37 °C for 1 h in the dark, so that the disulfide bond of the antibody was reduced to obtain a reaction solution 1.

3. Conjugation of antibody to linker-drug intermediate compound:

An appropriate amount of linker-payload was dissolved in 50% aqueous acetone solution to allow the final concentration to be 10 mg/mL; solution I was added to the reaction solution 1 (solution I:reaction solution 1 (v/v) = 1:10), the mixture was well mixed, and then an appropriate amount of the above linker-payload solution dissolved in the aqueous acetone solution was added to make the molar weight of the linker-payload be N3; the reaction mixture was shaken at 22 °C for 1 h in the dark to obtain a reaction solution 2.

4. Termination of conjugation:

An ultrafiltration centrifuge tube was wet using solution L; the reaction solution 2 was subjected to ultrafiltration using 20 times volume of solution L and 20 times volume of solution G successively, an appropriate amount of solution J and solution K was added, and the mixture was cryopreserved at -80 °C.

The experimental conditions and groups are shown in Table 1-1 below.

Table 1-1. Experimental conditions and groups

| ADC | Antibody | linker-payload | N1:N2 | N1:N3 |
|---|---|---|---|---|
| Patritumab-eribulin-D2 | Patritumab | MC-GGFG-eribulin | 1:2 | 1:3 |
| Patritumab-eribulin-D8 | Patritumab | MC-GGFG-eribulin | 1:8.5 | 1:10.5 |
| Patritumab-DDDXd-D8 | Patritumab | MC-GGFG-DDDXd | 1:8.5 | 1:10.5 |

(2) A patritumab-eribulin conjugate with DAR of 4.0-4.2 was prepared according to the following experimental procedures and named patritumab-eribulin-D4:

**Experimental procedures:**

1. Buffer exchange of antibody:

A 30 KD ultrafiltration centrifuge tube was fully wet using solution G, the antibody was buffer-exchanged into solution G, and an appropriate amount of solution G was added to adjust the concentration of the antibody to 10 mg/mL; then, an appropriate amount of solution M was added to adjust the pH of the antibody solution to about 7.0.

2. Antibody reduction:

The molar weight of the antibody was calculated and recorded as N1; an appropriate amount of solution H was added into the antibody solution to ensure that the molar weight of TCEP in the reaction system was N2; the resulting mixture was reacted at 5-10 °C for 6 h in the dark, so that the disulfide bond of the antibody was reduced to obtain a reaction solution 3.

3. Conjugation of antibody to linker-drug intermediate compound:

An appropriate amount of linker-payload was dissolved in 50% aqueous acetone solution to allow the final concentration to be 10 mg/mL; an appropriate amount of the above linker-payload solution dissolved in the aqueous acetone solution was added to the reaction solution 3 to make the molar weight of the linker-payload be N3; the reaction mixture was reacted at 5-10 °C for 40 min in the dark to obtain a reaction solution 4.

4. Termination of conjugation:

An ultrafiltration centrifuge tube was wet using solution L; the reaction solution 4 was subjected to ultrafiltration using 20 times volume of solution L and 20 times volume of solution G successively, an appropriate amount of solution J and solution K was added, and the mixture was cryopreserved at -80 °C.

The experimental conditions and groups are shown in Table 1-2 below.

Table 1-2. Experimental conditions and groups

| ADC | Antibody | linker-payload | N1:N2 | N1:N3 |
|---|---|---|---|---|
| Patritumab-eribulin-D4 | Patritumab | MC-GGFG-eribulin | 1:2.58 | 1:5.1 |

**Example 5: Determination of DAR Values of Antibody-Drug Conjugates**

Components of the patritumab-eribulin conjugates prepared in Example 4 above were isolated by using butyl-bonded nonporous polystyrene/divinylbenzene (PS/DVB) packing. The hydrophobic property of the protein molecules was improved using a neutral high-salt mobile phase, thereby allowing binding to the hydrophobic bonds in the chromatographic column, and then the substances were eluted by gradually decreasing the salt concentration and gradually increasing the proportion of isopropanol, with the less hydrophobic components eluted first and the more hydrophobic components eluted later.

The specification of the chromatographic column was Sepax HIC-Butyl, 4.6 × 100 mm, 5 μm, and the column temperature was 25 °C. The mobile phase A was 10 mM phosphate buffered saline-1.5 M ammonium sulfate at pH 7.0 (1.42 g of anhydrous disodium hydrogen phosphate and 198.21 g of ammonium sulfate were weighed out, added to about 800 mL of ultrapure water, and dissolved completely by stirring, then the pH was adjusted to 7.0±0.1 with phosphoric acid, the volume was brought to 1 L, and the mixture was mixed well and filtered through a 0.22 μM filter membrane). The mobile phase B was 10 mM phosphate buffered saline at pH 7.0 (1.42 g of anhydrous disodium hydrogen phosphate was weighed out, added to about 800 mL of ultrapure water, and dissolved completely by stirring, then the pH was adjusted to 7.0±0.1 with phosphoric acid, the volume was brought to 1 L, and the mixture was mixed well and filtered through a 0.22 μM filter membrane). Mobile phase C was 100% isopropanol. The flow rate was 0.5 mL/min, gradient elution was carried out for 30 min, and the parameters for mobile phase were as follows: from 75% mobile phase A plus 25% mobile phase B to 75% mobile phase B plus 25% mobile phase C in 0-15 min, 75% mobile phase B plus 25% mobile phase C in 15-20 min, 75% mobile phase A plus 25% mobile phase B in 20-30 min. The patritumab-eribulin conjugates were each subjected to 2-fold dilution with the initial mobile phase at 0 min to obtain test solutions, the loading volume was adjusted according to the concentration of the patritumab-eribulin conjugates, and 50 μg of protein was loaded. The absorbance values at 280 nm wavelength were detected.

Data were processed, and the results were quantitatively analyzed using the area normalization method. The percentages of ADC peak areas for containing 0, 1, 2, 3, 4, 5, 6, 7, and 8 cytotoxic drugs were calculated separately, and DAR values were calculated. The calculation formula is as follows: DAR value = (percentage of ADC peak area for containing 0 cytotoxic drugs × 0 + percentage of ADC peak area for containing 1 cytotoxic drug × 1 + percentage of ADC peak area for containing 2 cytotoxic drugs × 2 + percentage of ADC peak area for containing 3 cytotoxic drugs × 3 + percentage of ADC peak area for containing 4 cytotoxic drugs × 4 + percentage of ADC peak area for containing 5 cytotoxic drugs × 5 + percentage of ADC peak area for containing 6 cytotoxic drugs × 6 + percentage of ADC peak area for containing 7 cytotoxic drugs × 7 + percentage of ADC peak area for containing 8 cytotoxic drugs × 8)/100%.

The patritumab-eribulin conjugates were prepared and assayed by the methods in Examples 4 and 5, respectively, and had a structure shown below:

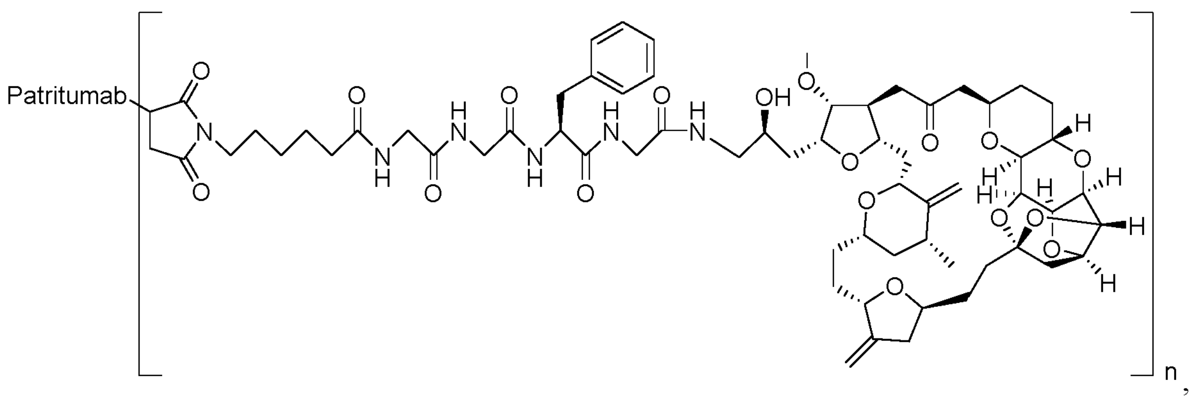

wherein, the determined DAR value of patritumab-eribulin-D2 was 2.6; the determined DAR value of patritumab-eribulin-D4 was 4-4.2; the determined DAR value of patritumab-eribulin-D8 was 7.6.

Patritumab-DDDXd-D8 was prepared and assayed by the methods in Examples 4 and 5, respectively, and had a structure shown below:

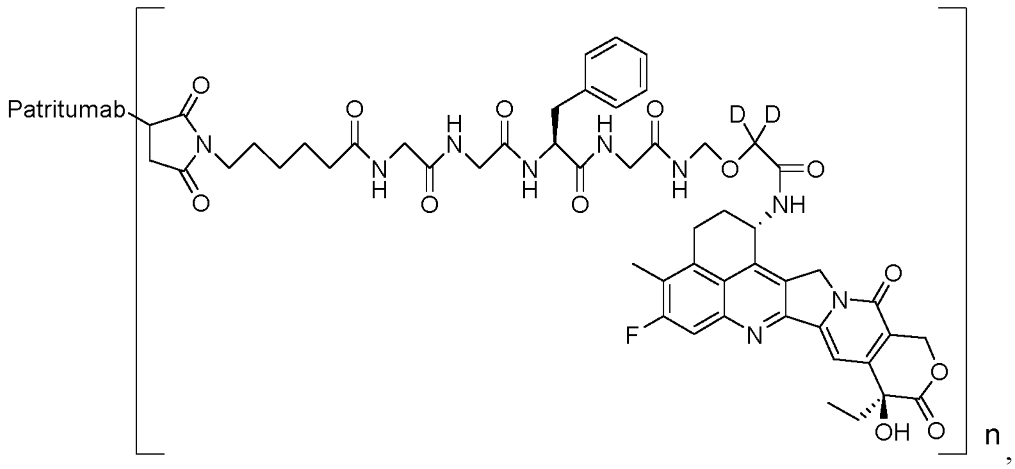

wherein the determined DAR value was 7.9.

**Example 6: Aggregate Verification for Antibody-Drug Conjugates**

Components of the patritumab-drug conjugates prepared in Example 4 above were isolated using a gel chromatographic column. Elution was performed using a neutral buffer containing 10% isopropanol as the mobile phase, and the components were eluted out in descending order according to their molecular weights. The gel chromatographic column used was an ACQUITY UPLC Protein BEH SEC Column 200Å, 1.7 μm, 4.6 × 300 mm, and the column temperature was 25 °C. The mobile phase was 50 mM phosphate buffered saline-200 mM sodium chloride-10% isopropanol at pH 7.0 (12.53 g of disodium hydrogen phosphate dodecahydrate, 2.33 g of sodium dihydrogen phosphate dihydrate, and 11.69 g of sodium chloride were weighed out, added to about 800 mL of ultrapure water, and dissolved completely by stirring, and ultrapure water was added to bring the volume to 1000 mL for use; 100 mL of isopropanol was added the above solution to bring the volume to 1000 mL, and the mixture was well mixed and filtered through a 0.22 μm filter). 20 μg of the patritumab-drug conjugate was weighed out and injected into a liquid chromatograph, and the detection at the wavelength of 280 nm was carried out. The flow rate was 0.3 mL/min, and isocratic elution was performed for 15 min.

Data were processed, and the results were quantitatively analyzed using the area normalization method. The peak area percentages for the aggregate, immunoglobulin monomer, and low molecular weight impurity were calculated. The aggregate peak appeared before the main peak, which represents the immunoglobulin monomer, and the low molecular weight impurity peaks appeared after the main peak. The content percentages of monomer, aggregate, and low molecular weight impurity of the patritumab-drug conjugates are shown in Table 2.

Table 2. Content of monomer, aggregate and low molecular weight impurity of patritumab-drug conjugates

| Name | Patritumab-eribul in-D2 | Patritumab-crib ulin-D4 | Patritumab-eribul in-D8 | Patritumab-DDD Xd-D8 |
|---|---|---|---|---|
| Aggregate | 0.29% | 0.52% | 0% | 0.95% |
| Monomer | 98.36% | 97.85% | 97.44% | 97.55% |
| Low molecular weight fragment | 1.35% | 1.62% | 2.56% | 1.5% |

**Example 7: Cell Binding Activities of Antibody-Drug Conjugates**

Based on the FACS method and by using patritumab-DDDXd-D8 and patritumab as controls, the patritumab-eribulin conjugates prepared in Example 4 above were analyzed for binding activity to cells with different HER3 expression levels, including MCF-7, HCC1569, and BT474 cells with high HER3 expression level, MDA-MB-468 and JIMT-1 cells with medium HER3 expression level, SW620 cells with low HER3 expression level, and HER3-negative A549 cells.

1 × $10^5$ cells were added to each well of a 96-well cell culture plate, and the patritumab-eribulin conjugates

were each diluted at an initial concentration of 135.14 nM (4-fold dilution, 9 concentration gradients) using a FACS buffer (Miltenyi Biotec, Cat No.: 130-091-221). The cells were incubated at 4 °C for 60 min and centrifuged at 1000 rpm for 5 min. The supernatant was discarded, the cells were washed 3 times with pre-cooled PBS (pH 7.4), 1:200 (v/v) diluted goat anti-human IgG-Fcy-PE secondary antibody (Jackson immunoresearch, Cat. No.: 109-116-170) was added at 100 μL/well, and the cells were then incubated at 4 °C for 30 min. The cells were washed 3 times with pre-cooled PBS (pH 7.4) and resuspended in 100 μL of PBS (pH 7.4), and then the fluorescence signals were analyzed and detected using a flow cytometer (Sartorius, iQUE). The binding activities of the patritumab-eribulin conjugates and the controls to each of the above cells were measured by mean fluorescence intensity (MFI) of staining. Data were analyzed using GraphPad Prism5. The results are shown in FIGs. 1A-1G, and the calculated $EC_{50}$ values are shown in Table 3 below. The results show that the binding activities of the patritumab-eribulin conjugates with different DAR values to cells with high, medium, and low HER3 expression levels are equivalent to that of patritumab, and the patritumab-eribulin conjugates do not bind to HER3-negative A549 cells.

Table 3. Binding activities of patritumab-eribulin conjugates to cells with different HER3 expression levels

| | $EC_{50}$ (nM) | | | | | |
|---|---|---|---|---|---|---|
| | MCF-7 | BT474 | HCC1569 | MDA-MB-468 | JIMT-1 | SW620 |
| Patritumab-eribulin-D2 | 0.8554 | 3.049 | 1.416 | 1.564 | 1.588 | 1.888 |
| Patritumab-eribulin-D4 | 0.9318 | 2.776 | 1.486 | 1.671 | 1.04 | 1.28 |
| Patritumab-eribulin-D8 | 1.019 | 2.854 | 1.487 | 3.307 | 1.729 | 1.744 |
| Patritumab-DDDXd-D8 | 0.7703 | 2.68 | 1.164 | 1.7 | 1.304 | 1.37 |
| Patritumab | 0.807 | 3.017 | * | 1.299 | 1.515 | 1.465 |
| *: Not detected. | | | | | | |

**Example 8: Endocytosis Assay of Antibody-Drug Conjugates**

Based on the FACS method and by using patritumab-DDDXd-D8 and patritumab as controls, the patritumab-eribulin conjugates prepared in Example 4 above were analyzed for endocytosis in cells with different HER3 expression levels, including MCF-7 and BT474 cells with high HER3 expression level, NCI-N87 cells with medium HER3 expression level, and SW620 cells with low HER3 expression level.

The cell density was adjusted to $1 \times 10^6$ cells/mL and the cells were added to a 96-well cell culture plate at 50 μL/well. Sample preparation: the patritumab-eribulin conjugates were each diluted to a concentration of 20 μg/mL and labeled as S1, and then subjected to 3-fold gradient dilution to obtain 9 samples S1-S9; the sample solution obtained after gradient dilution was added to the cell culture plate at 50 μL/well, and the mixture was incubated at 4 °C for 30 min. After the incubation, the 96-well cell culture plate was taken out, the cells were centrifuged at 400 g for 4 min at 4 °C, and the supernatant was discarded. A 1:200 (v/v) diluted pHrodo™ Green Maleimide (green maleimide; Invitrogen, Cat No.: P35370)-labeled AffiniPure Goat Anti-Human IgG, Fcy fragment specific (goat anti-human IgG; Fc fragment specific antibody, Jackson Immuno; Cat No.: 109-005-190) was added at 50 μL/well and the cells were incubated at 4 °C. After 30 min, the cells were washed, and the cell culture medium was added at 50 μL/well. The mixture was well mixed, internalized for 2 h at 37 °C, and placed in a flow cytometer (Sartorius, iQUE), and the fluorescence reading value of a BL1 channel was measured. Data were analyzed using GraphPad Prism5. The results are shown in FIGs. 2A-2D, and the calculated $EC_{50}$ values are shown in Table 4 below. The results show that the patritumab-eribulin conjugates with different DAR values show significant endocytosis in cells with high and medium HER3 expression levels, and the endocytosis is relative weak in SW620 cells with low expression level.

Table 4. Endocytosis of patritumab-eribulin conjugates in cells with different HER3 expression levels

| | $EC_{50}$ (nM) | | |
|---|---|---|---|
| | MCF-7 | BT474 | NCI-N87 |
| Patritumab-eribulin-D2 | 0.5801 | 1.07 | 4.495 |
| Patritumab-eribulin-D4 | 0.7968 | 0.9615 | 4.721 |
| Patritumab-eribulin-D8 | 1.255 | 0.9306 | 9.463 |

(continued)

| | $EC_{50}$ (nM) | | |
|---|---|---|---|
| | MCF-7 | BT474 | NCI-N87 |
| Patritumab-DDDXd-D8 | 0.869 | 0.8587 | 56.82 |
| Patritumab | 0.5088 | 0.8208 | ~4.479E18 |

**Example 9: Cell Killing Activities of Antibody-Drug Conjugates**

To examine the proliferation inhibition effect of the patritumab-eribulin conjugates prepared in Example 4 above against tumor cells, cells with different HER3 expression levels were used for the killing activity assay, including BT474, MCF-7, and HCC1569 cells with high HER3 expression level, SKBR3, NCI-N87, JIMT-1, and MDA-MB-468 cells with medium HER3 expression level, and SW620 and WiDr cells with low HER3 expression level, and the patritumab-DDDXd-D8 was used as a control.

Cells in the logarithmic growth phase were added to a 96-well plate at 100 μL/well, and the cell density was 1 × $10^4$/mL or 2 × $10^4$/mL. Adherence culture was carried out at 37 °C and 5% COz overnight. Sample preparation: the patritumab-eribulin conjugates were each formulated into test samples with a basal medium containing 10% FBS (initial concentration of 5 μg/mL, 5-fold gradient dilution, nine gradients). Cells subjected to overnight adherence culture were taken out. For the experimental groups, the diluted test sample was added at 50 μL/well, and for the control group, the basal medium containing 10% FBS was added at 50 μL/well. The cells were then cultured for 96 h, 120 h, or 144 h, and then a CellTiter-Glo Luminescent Cell kit (Promega, Cat No.: G7572) was used for assay. The 96-well plate was taken out, the CTG detection solution (Promega, Cat No.: G7572) was added at 75 μL/well, and the mixture was well mixed by shaking and incubated at room temperature in the dark for 10 min. Then 180 μL of solution was pipetted from each well and transferred to an opaque white plate, bubbles were removed, chemiluminescence values were read and killing rates were calculated.

Killing rate (%) = (1 - chemiluminescence value of experimental group/chemiluminescence value of control group) × 100%.

Data were analyzed using Graphpad Prism5. The results are shown in FIGs. 3A-3I, and the calculated $EC_{50}$ values are shown in Tables 5-1 and 5-2 below. The results show that for cells with high, medium, and low HER3 expression levels, the larger the DAR value is, the stronger the killing activity of the patritumab-eribulin conjugate is, and the killing activities of the patritumab-eribulin conjugates with different DAR values are superior to that of patritumab-DDDXd-D8.

Table 5-1. Killing activities of patritumab-eribulin conjugates against cells with high HER3 expression level

| | $EC_{50}$ (nM) | | |
|---|---|---|---|
| | BT474 | MCF-7 | HCC1569 |
| Patritumab-eribulin-D2 | 9.345E-02 | 1.173 | 3.600 |
| Patritumab-eribulin-D4 | 3.982E-02 | 9.942E-02 | 7.780E-01 |
| Patritumab-eribulin-D8 | 1.807E-02 | 3.613E-02 | 2.457E-01 |
| Patritumab-DDDXd-D8 | 1.045E+01 | / | 1.829E+01 |

Table 5-2. Killing activities of patritumab-eribulin conjugates against cells with medium HER3 expression level

| | $EC_{50}$ (nM) | | | |
|---|---|---|---|---|
| | SKBR3 | NCI-N87 | JIMT-1 | MDA-MB-468 |
| Patritumab-eribulin-D2 | 1.937E-01 | / | 1.047E+01 | 1.209E+01 |
| Patritumab-eribulin-D4 | 4.299E-02 | 1.386E+01 | 3.266E+00 | 1.413E+00 |
| Patritumab-eribulin-D8 | 2.319E-02 | 6.905E-01 | 9.462E-01 | 3.206E-01 |

(continued)

| | $EC_{50}$ (nM) | | | |
|---|---|---|---|---|
| | SKBR3 | NCI-N87 | JIMT-1 | MDA-MB-468 |
| Patritumab-DDDXd-D8 | 5.785E+01 | 3.709E-01 | / | / |

**Example 10. Pharmacodynamic Evaluation of Antibody-Drug Conjugates in Nude Mouse Subcutaneous Xenograft Tumor Models of JIMT-1 Human Breast Cancer Cells**

The *in vivo* efficacy of the patritumab-eribulin conjugates prepared in Example 4 above was evaluated by the nude mouse subcutaneous xenograft tumor models of JIMT-1 human breast cancer cells, a trastuzumab-resistant cell strain.

SPF-grade female nude mice (from Changzhou Cavens Laboratory Animal Ltd.) were inoculated subcutaneously at the right side armpit with $2 \times 10^6$ JIMT-1 cells per mouse. When the mean tumor volume reached 100-300 $mm^3$, the animals were divided into 5 groups of 6, and the specific grouping and administration regimen are shown in Table 6.

Table 6. Grouping and administration regimen

| Group | Drug | Dose (mg/kg) | Route of administration | Frequency of administration |
|---|---|---|---|---|
| 1 | Vehicle control | N/A | Tail vein injection | Q1W |
| 2 | Patritumab-DDDXd-D8 | 3 | Tail vein injection | Q1W |
| 3 | Patritumab-eribulin-D2 | 3 | Tail vein injection | Q1W |
| 4 | Patritumab-eribulin-D4 | 3 | Tail vein injection | Q1W |
| 5 | Patritumab-eribulin-D8 | 3 | Tail vein injection | Q1W |
| Q1W: once weekly. | | | | |

The day of grouping was d0, and the tail vein administration was performed at d1 after grouping. The tumor volume was measured 2-3 times a week, and meanwhile, the mice were weighed and the data were recorded; the general behavior of the mice was observed and recorded every day. After the experiment was completed, the tumors were removed, weighed, and photographed.

The detection indexes include:

$$\text{Tumor volume TV } (mm^3) = 1/2 \times (a \times b^2)$$

(wherein a is the long diameter and b is the short diameter);

$$\text{Relative Tumor Volume RTV} = TV_t/TV_0,$$

wherein $TV_0$ is the tumor volume at d0, and $TV_t$ is the tumor volume at each measurement;

$$\text{Relative tumor proliferation rate T/C (\%)} = (T_{RTV}/C_{RTV}) \times 100\%,$$

wherein $T_{RTV}$ is RTV of treatment group, and $C_{RTV}$ is RTV of control group.

$$\text{Tumor growth inhibition rate: } 1 - T/C;$$

$$\text{Tumor inhibition rate TGI (\%)} = (1 - TW/TW_0) \times 100\%;$$

wherein, TW is the tumor weight of the treatment group, and $TW_0$ is the tumor weight of the control group;

$$\text{Weight change rate WCR (\%)} = (Wt_t - Wt_0)/Wt_0 \times 100\%,$$

wherein $Wt_0$ is the body weight of animal at d0, and $Wt_t$ is the body weight of animal at each measurement.

The effect of each drug on the tumor volume, tumor weight, and body weight of mouse is shown in FIGs. 4-6, and the results of the detection indexes are shown in Table 7 below. When the experiment ended on d21, no animals died, the weight increase of the mice in each treatment group was equivalent to that in the model group, the drugs showed no significant toxic effect and the safety was good. The patritumab-eribulin conjugates with different DAR values show good *in vivo* tumor proliferation inhibition activity and are superior to patritumab-DDDXd-D8; the larger the DAR value of the patritumab-eribulin conjugate is, the stronger the *in vivo* tumor proliferation inhibition activity is.

Table 7. Tumor inhibition effect of patritumab-eribulin conjugates in nude mouse xenograft tumor models of JIMT-1 human breast cancer cells

| Group | Tumor growth inhibition rate 1 - T/C | Tumor inhibition rate TGI |
|---|---|---|
| 2 | 24.4% | 26.6% |
| 3 | 36.6% | 41.1% |
| 4 | 65.9% | 68.9% |
| 5 | 82.9% | 84.3% |

According to the content disclosed in the present disclosure, the methods of the present disclosure have been described in terms of preferred embodiments. However, for those skilled in the art, changes may be applied to the methods and the steps or the sequence of steps of the methods described herein without departing from the concept, spirit and scope of the present disclosure.

The disclosed contents of all documents cited herein are hereby incorporated by reference to the extent that they provide exemplary, procedural and additional details supplementary to those described herein.

## Claims

1. An antibody-drug conjugate having general formula $Ab\text{-}(L\text{-}U)_n$ or a pharmaceutically acceptable salt or a solvate thereof, wherein Ab represents an antibody moiety, L represents a linker moiety, U represents a cytotoxic drug moiety, and n is an integer or a decimal selected from numbers from 1 to 10, wherein the antibody-drug conjugate comprises a structure of formula IIa below:

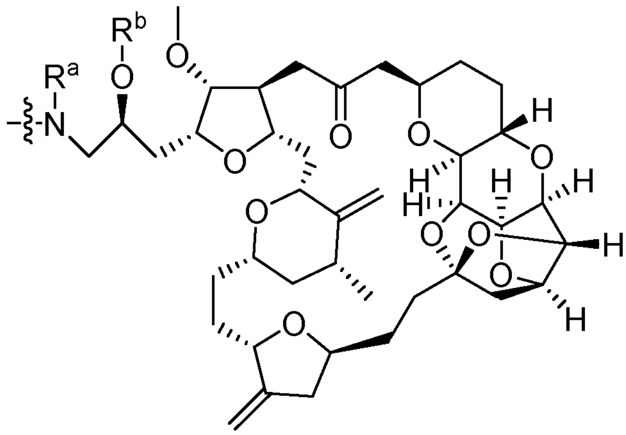

IIa,

wherein,

$R^a$ is selected from a hydrogen atom, a deuterium atom, optionally substituted $C_{1-6}$ alkyl, optionally substituted

$C_{3-7}$ cycloalkyl, optionally substituted $C_{3-7}$ heterocyclyl, optionally substituted $C_{6-10}$ aryl, and optionally substituted $C_{5-12}$ heteroaryl;

$R^b$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;

or,

$R^a$ and $R^b$, together with an atom to which they are attached, form optionally substituted 5-8 membered heterocyclyl.

**2.** The antibody-drug conjugate or the pharmaceutically acceptable salt or the solvate thereof according to claim 1, wherein the antibody-drug conjugate comprises a structure of formula IIIa below:

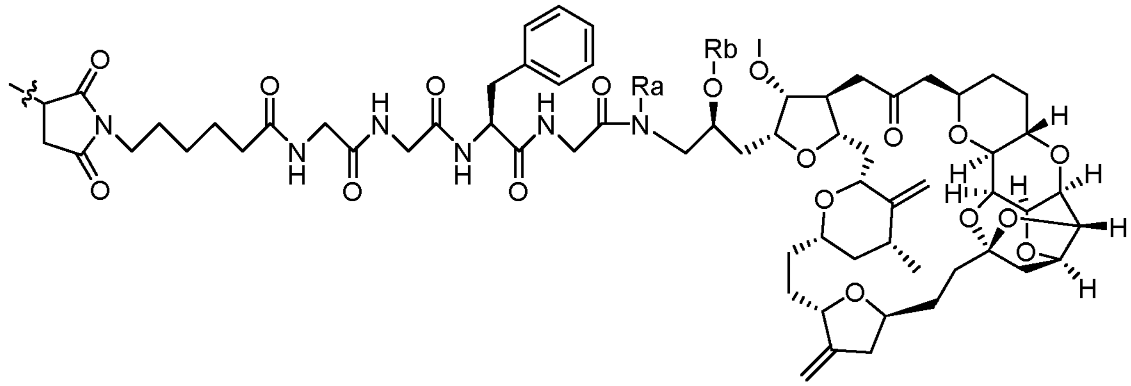

IIIa.

**3.** The antibody-drug conjugate or the pharmaceutically acceptable salt or the solvate thereof according to claim 1 or 2, wherein the antibody-drug conjugate is a structure of formula IV below:

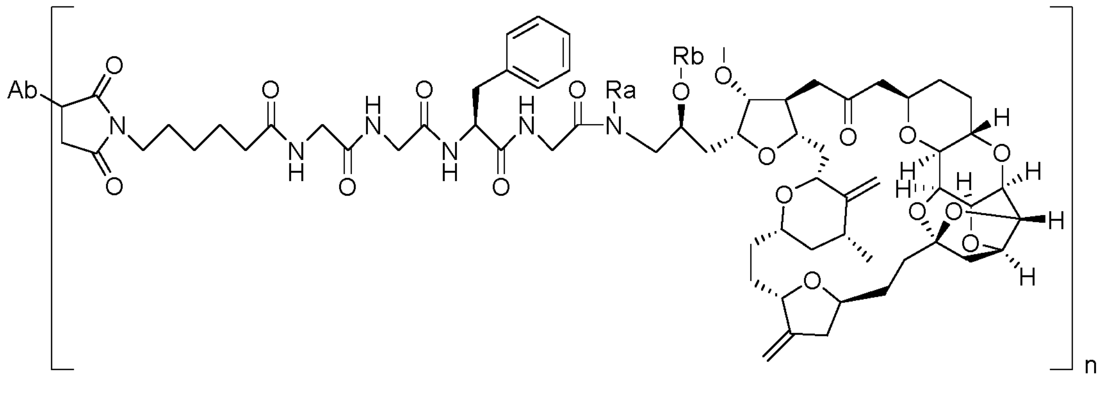

IV,

wherein,

Ab represents an antibody moiety, and
n is an integer or a decimal selected from numbers from 1 to 10.

**4.** The antibody-drug conjugate or the pharmaceutically acceptable salt or the solvate thereof according to any one of claims 1-3, wherein $R^a$ and $R^b$ are each independently selected from a hydrogen atom, methyl, ethyl, propyl, and isopropyl.

**5.** The antibody-drug conjugate or the pharmaceutically acceptable salt or the solvate thereof according to claim 1 or 2, wherein the antibody-drug conjugate comprises a structure of formula IIIa-1 below:

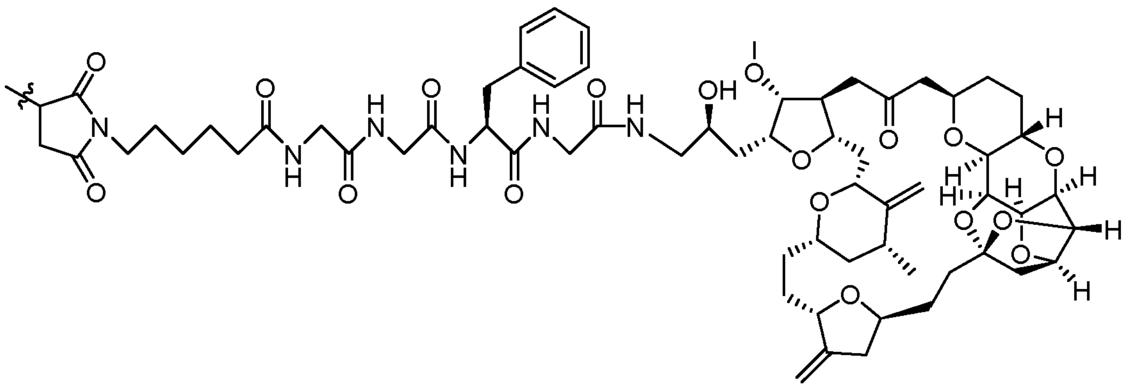

IIIa-1.

6. The antibody-drug conjugate or the pharmaceutically acceptable salt or the solvate thereof according to claim 3, wherein the antibody-drug conjugate is a structure of formula IV-1 below:

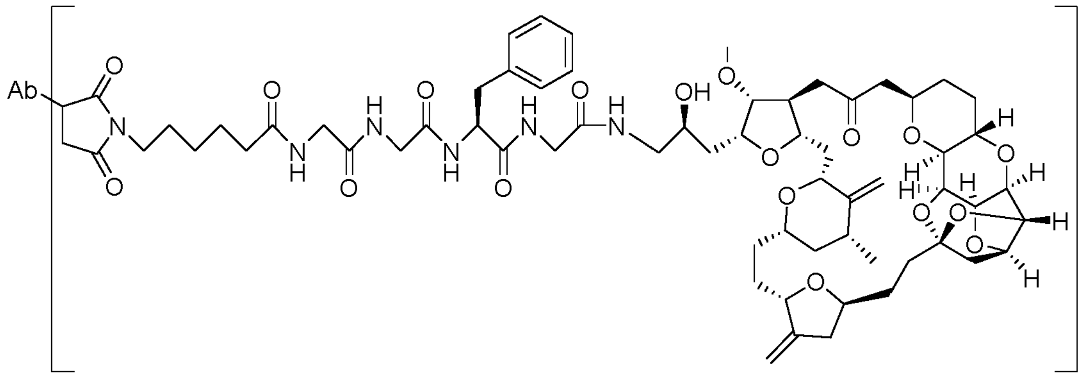

IV-1.

7. The antibody-drug conjugate or the pharmaceutically acceptable salt or the solvate thereof according to any one of claims 1-6, wherein n is 2-4.8, 2.6-4.8, 3.5-4.8, 4-4.8, 2-4.5, 2.6-4.5, 3.5-4.5, 4-4.5, 3.5-4.2, 3.5-4, 4-4.2, 7-8, 7-7.9, 7-7.6, 7-7.5, 7.1-8, 7.1-7.9, 7.1-7.6, 7.5-8, 7.6-8, or 7.6-7.9.

8. The antibody-drug conjugate or the pharmaceutically acceptable salt or the solvate thereof according to claim 7, wherein n is about 2.6, about 4, about 4.2, about 4.8, about 7, about 7.1, about 7.5, about 7.6, about 7.9, or about 8.

9. The antibody-drug conjugate or the pharmaceutically acceptable salt or the solvate thereof according to any one of claims 1-8, wherein Ab is an anti-HER3 antibody or an antigen-binding fragment thereof.

10. The antibody-drug conjugate or the pharmaceutically acceptable salt or the solvate thereof according to claim 9, wherein the anti-HER3 antibody or the antigen-binding fragment thereof comprises: HCDR1 comprising an amino acid sequence set forth in SEQ ID NO: 1, HCDR2 comprising an amino acid sequence set forth in SEQ ID NO: 2, HCDR3 comprising an amino acid sequence set forth in SEQ ID NO: 3, LCDR1 comprising an amino acid sequence set forth in SEQ ID NO: 4, LCDR2 comprising an amino acid sequence set forth in SEQ ID NO: 5, and LCDR3 comprising an amino acid sequence set forth in SEQ ID NO: 6.

11. The antibody-drug conjugate or the pharmaceutically acceptable salt or the solvate thereof according to claim 10, wherein the anti-HER3 antibody or the antigen-binding fragment thereof comprises a heavy chain variable region and a light chain variable region, wherein the heavy chain variable region comprises an amino acid sequence having at least 80% identity to an amino acid sequence set forth in SEQ ID NO: 7 and the light chain variable region comprises an amino acid sequence having at least 80% identity to an amino acid sequence set forth in SEQ ID NO: 8, or the heavy chain variable region comprises the amino acid sequence set forth in SEQ ID NO: 7 and the light chain variable region comprises the amino acid sequence set forth in SEQ ID NO: 8.

12. The antibody-drug conjugate or the pharmaceutically acceptable salt or the solvate thereof according to claim 10 or 11, wherein the anti-HER3 antibody or the antigen-binding fragment thereof comprises a heavy chain and a light

chain, wherein the heavy chain comprises an amino acid sequence having at least 80% identity to an amino acid sequence set forth in SEQ ID NO: 9, and the light chain comprises an amino acid sequence having at least 80% identity to an amino acid sequence set forth in SEQ ID NO: 10.

**13.** The antibody-drug conjugate or the pharmaceutically acceptable salt or the solvate thereof according to claim 9, wherein the anti-HER3 antibody is patritumab.

**14.** The antibody-drug conjugate or the pharmaceutically acceptable salt or the solvate thereof according to any one of claims 9-13, wherein the antibody-drug conjugate or the pharmaceutically acceptable salt or the solvate thereof exhibits one of or a combination of several of the following properties:

(a) binding to HER3;
(b) blocking binding of HER3 to a ligand;
(c) showing endocytosis in cells expressing HER3;
(d) having killing activity against HER3-expressing tumor cells; and
(e) having bystander effect.

**15.** A pharmaceutical composition, comprising the antibody-drug conjugate or the pharmaceutically acceptable salt or the solvate thereof according to any one of claims 1-14, wherein optionally, the pharmaceutical composition further comprises a pharmaceutically acceptable carrier.

**16.** Use of the antibody-drug conjugate or the pharmaceutically acceptable salt or the solvate thereof according to any one of claims 1-14 or the pharmaceutical composition according to claim 15 for preparing a medicament for treating cancer, wherein preferably, the cancer is HER3-positive cancer; preferably, the cancer is biliary tract cancer, carcinosarcoma, esophageal cancer, esophagogastric junction cancer, breast cancer, gastric cancer, pancreatic cancer, head and neck cancer, colorectal cancer, kidney cancer, cervical cancer, ovarian cancer, endometrial cancer, uterine cancer, melanoma, pharyngeal cancer, oral cancer, skin cancer, lung cancer, glioblastoma multiforme, glioblastoma, urothelial carcinoma, prostate cancer, bladder cancer, gastrointestinal stromal tumor, squamous cell carcinoma, peritoneal cancer, liver cancer, salivary gland carcinoma, vulvar cancer, thyroid cancer, testicular cancer, anal cancer, or penile cancer.

**17.** A method for treating cancer, comprising administering to a patient in need thereof a therapeutically effective amount of the antibody-drug conjugate or the pharmaceutically acceptable salt or the solvate thereof according to any one of claims 1-14 or the pharmaceutical composition according to claim 15, wherein preferably, the cancer is HER3-positive cancer; preferably, the cancer is biliary tract cancer, carcinosarcoma, esophageal cancer, esophagogastric junction cancer, breast cancer, gastric cancer, pancreatic cancer, head and neck cancer, colorectal cancer, kidney cancer, cervical cancer, ovarian cancer, endometrial cancer, uterine cancer, melanoma, pharyngeal cancer, oral cancer, skin cancer, lung cancer, glioblastoma multiforme, glioblastoma, urothelial carcinoma, prostate cancer, bladder cancer, gastrointestinal stromal tumor, squamous cell carcinoma, peritoneal cancer, liver cancer, salivary gland carcinoma, vulvar cancer, thyroid cancer, testicular cancer, anal cancer, or penile cancer.

**18.** The method according to claim 17, comprising contacting tumor cells with the antibody-drug conjugate or the pharmaceutically acceptable salt or the solvate thereof or the pharmaceutical composition, thereby killing the tumor cells or inhibiting growth of the tumor cells.

**19.** A linker-drug intermediate compound having a structure of formula III:

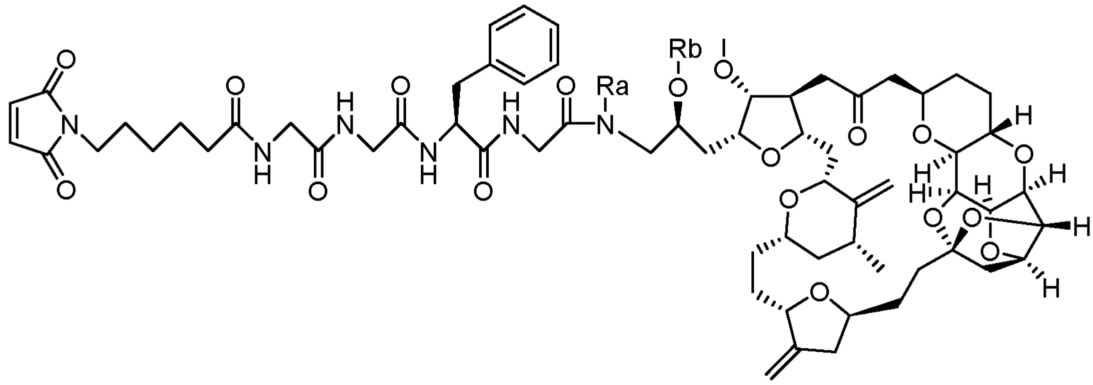

III,

wherein,

$R^a$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;

$R^b$ is selected from a hydrogen atom, a deuterium atom, optionally substituted $C_{1-6}$ alkyl, optionally substituted $C_{3-7}$ cycloalkyl, optionally substituted $C_{3-7}$ heterocyclyl, optionally substituted $C_{6-10}$ aryl, and optionally substituted $C_{5-12}$ heteroaryl;

or $R^a$ and $R^b$, together with an atom to which they are attached, form optionally substituted 5-8 membered heterocyclyl.

**20.** The linker-drug intermediate compound according to claim 19, wherein $R^a$ and $R^b$ are each independently selected from a hydrogen atom, methyl, ethyl, propyl, and isopropyl.

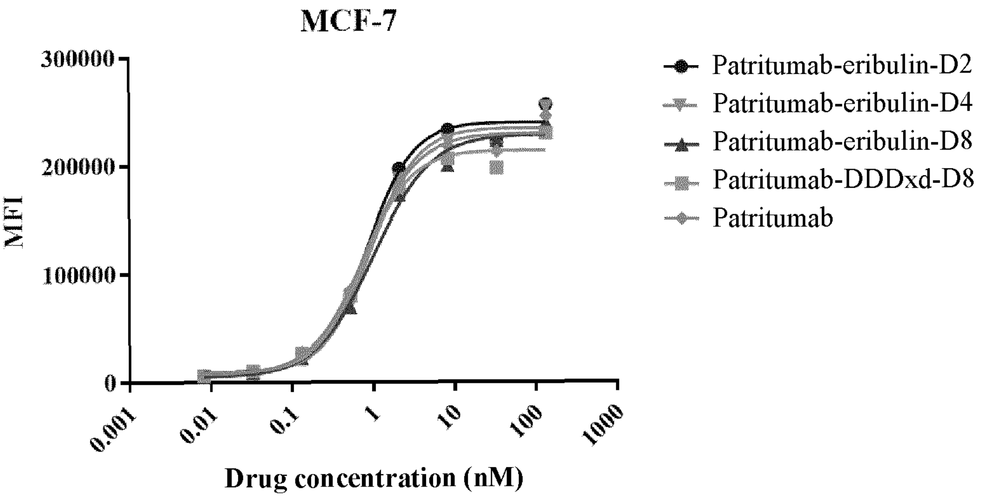


FIG. 1A

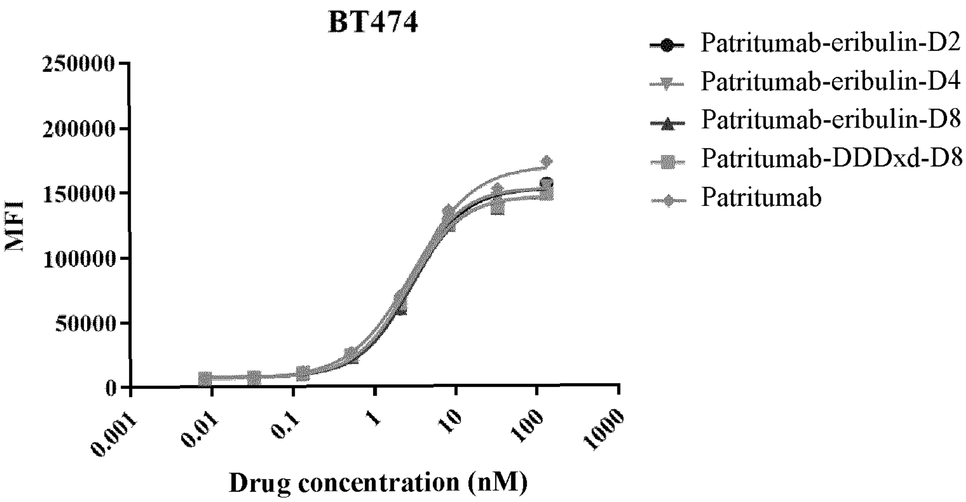


FIG. 1B

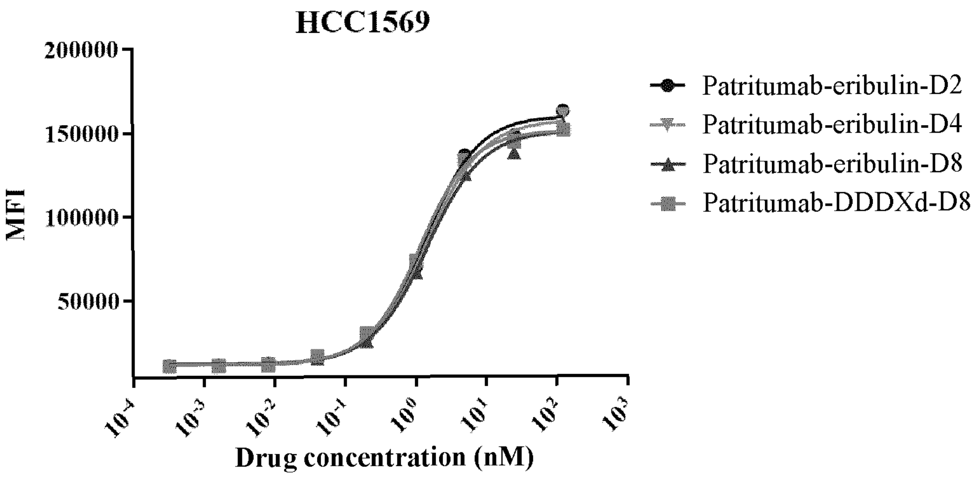


FIG. 1C

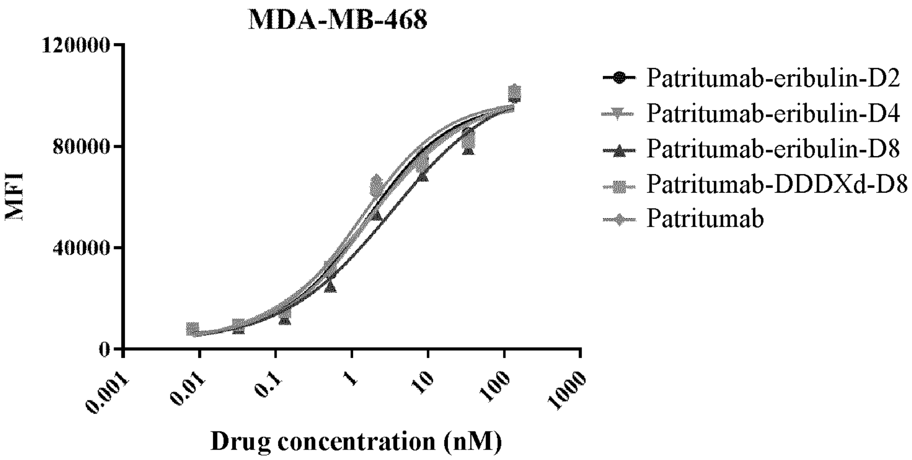


FIG. 1D

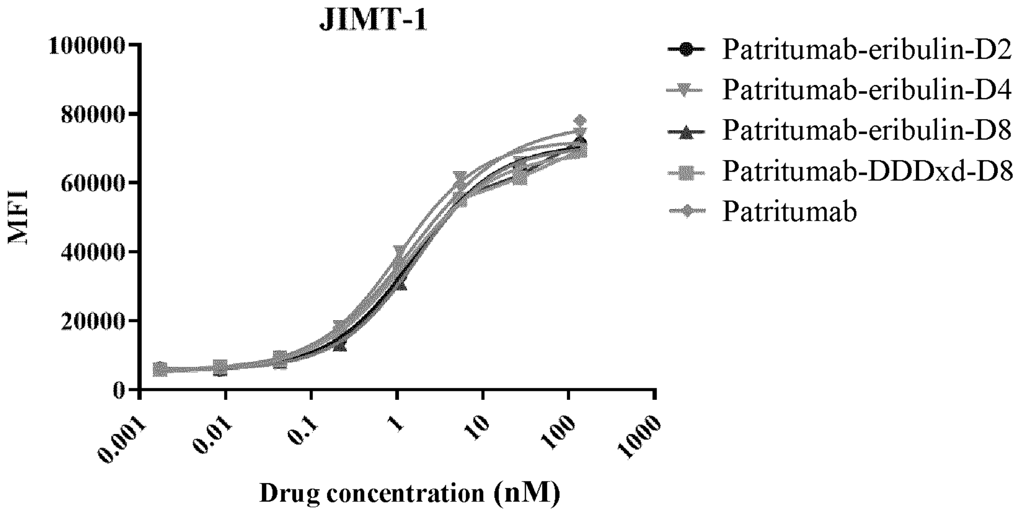


FIG. 1E

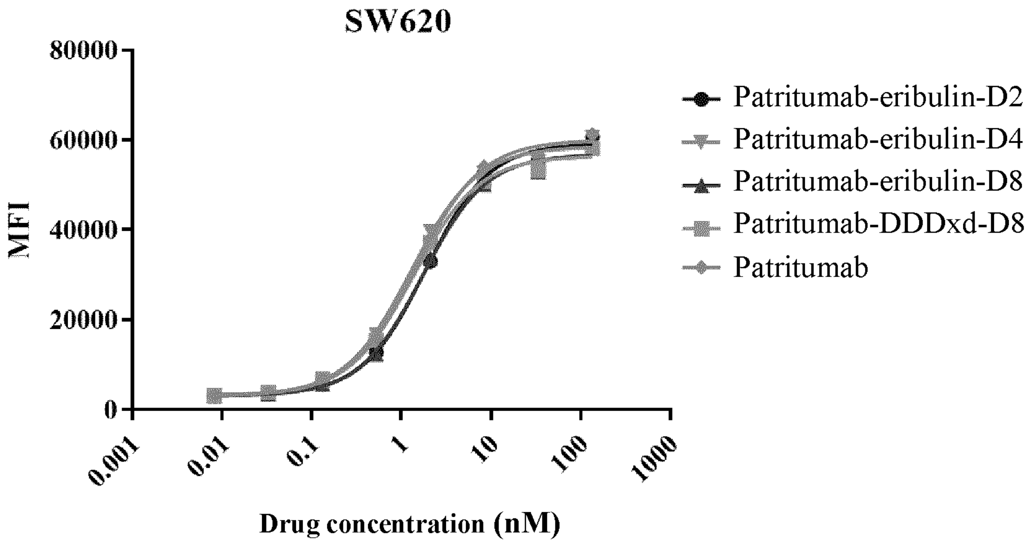


FIG. 1F

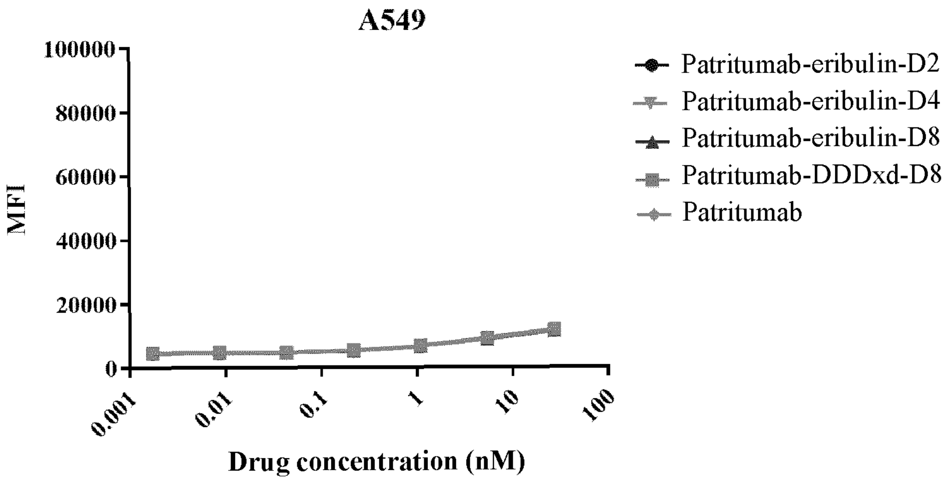


FIG. 1G

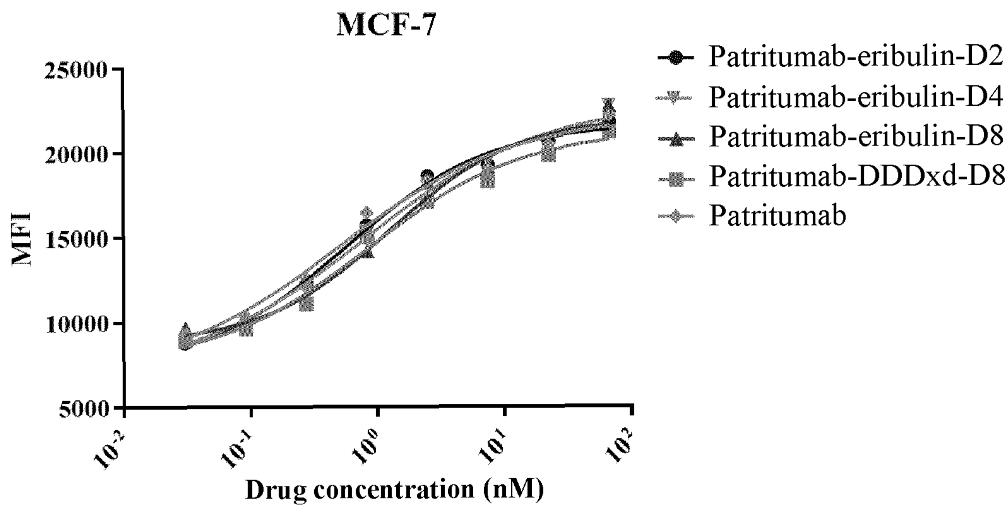


FIG. 2A

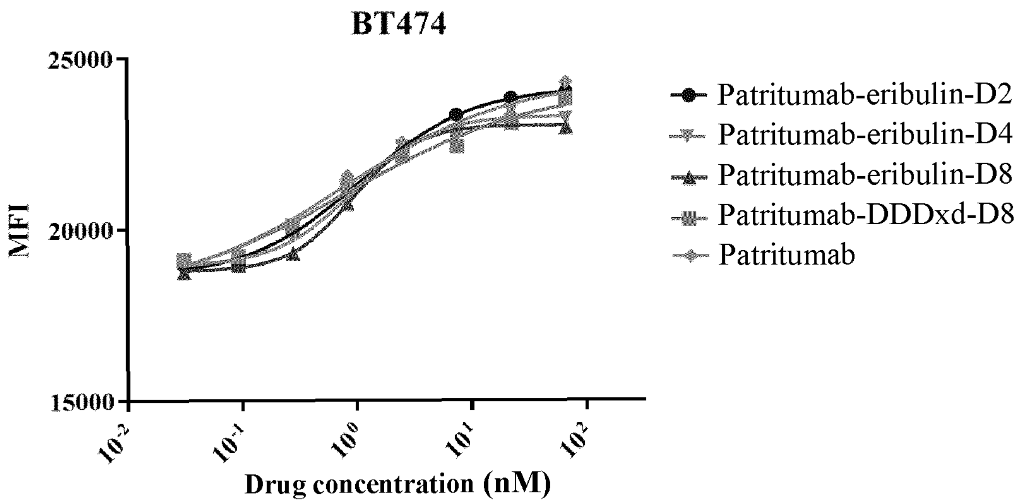


FIG. 2B

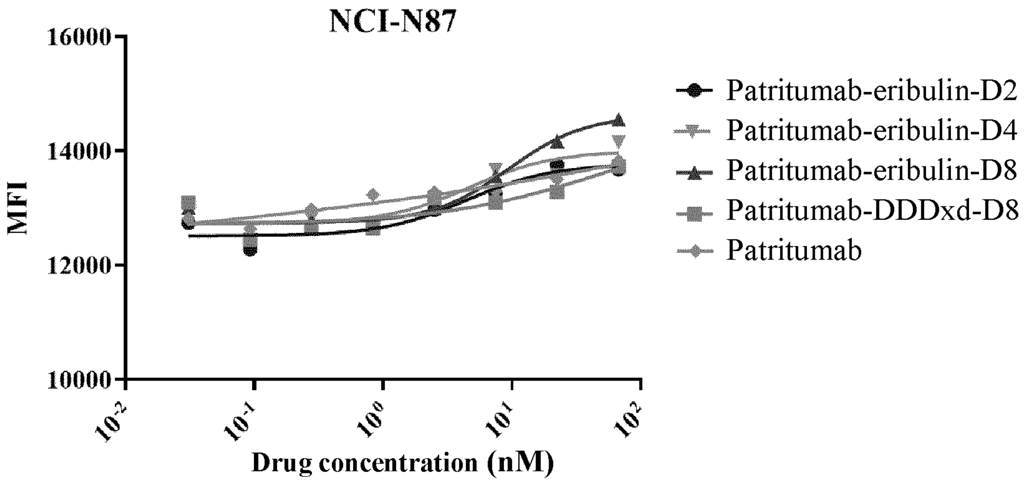


FIG. 2C

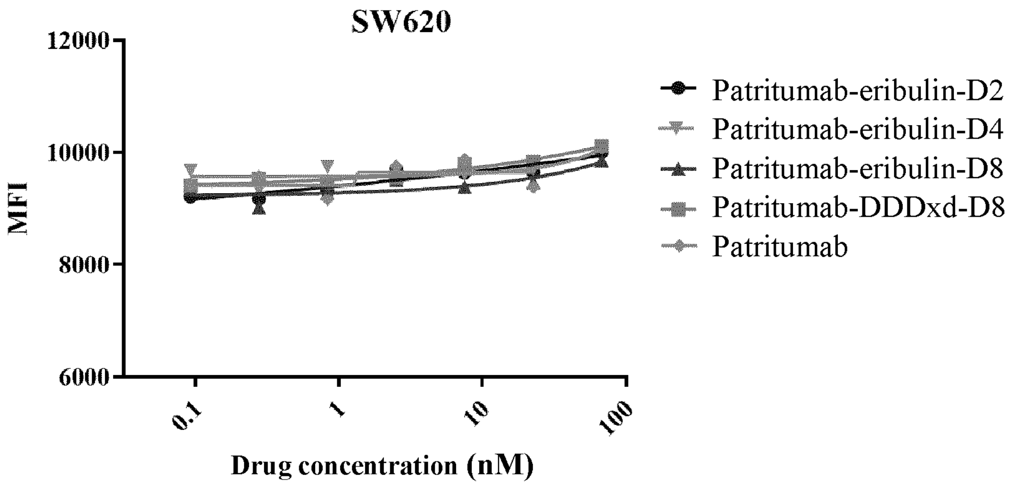


FIG. 2D

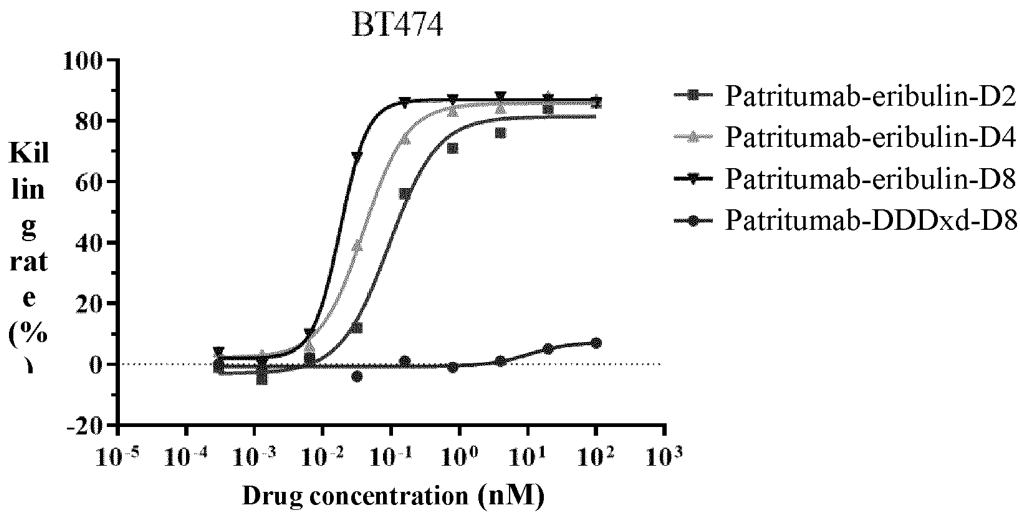


FIG. 3A

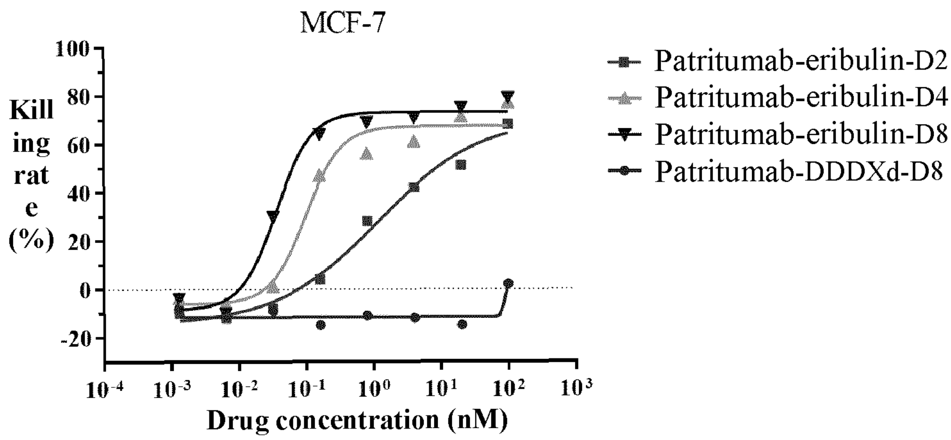


FIG. 3B

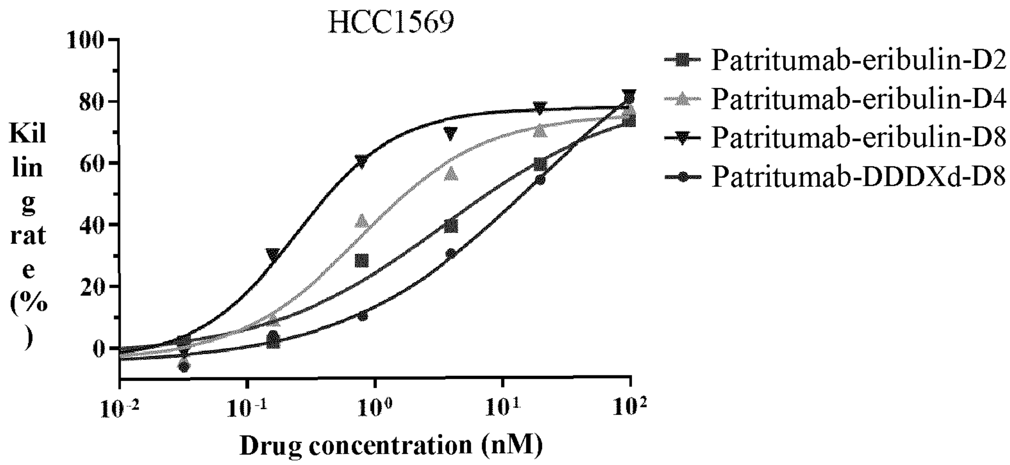


FIG. 3C

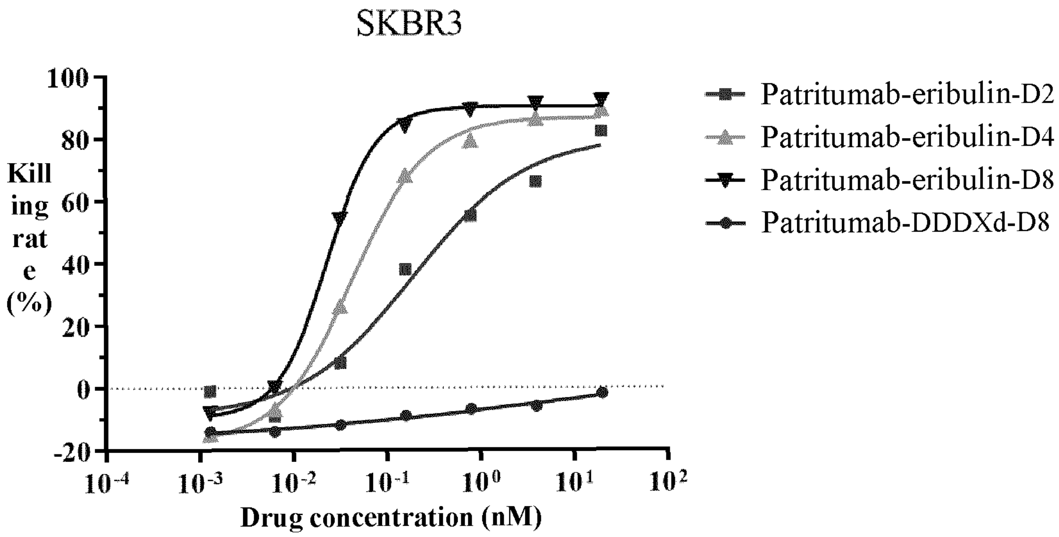


FIG. 3D

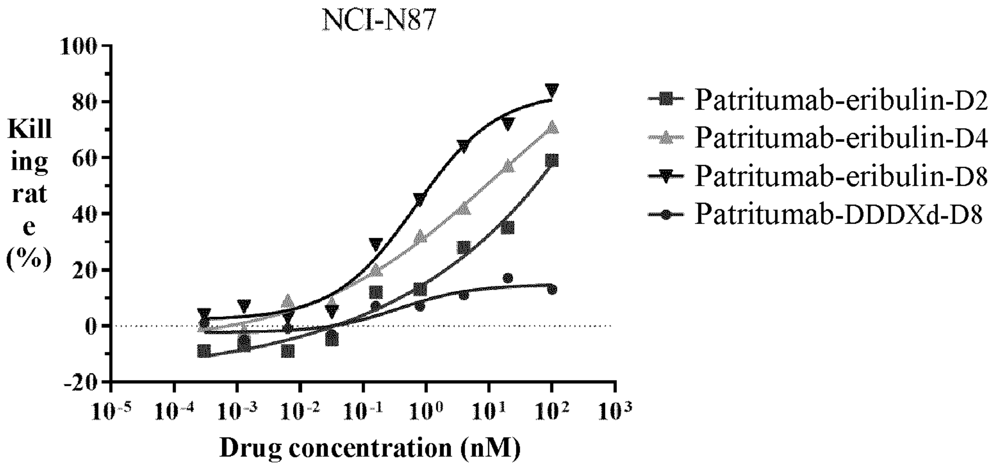


FIG. 3E

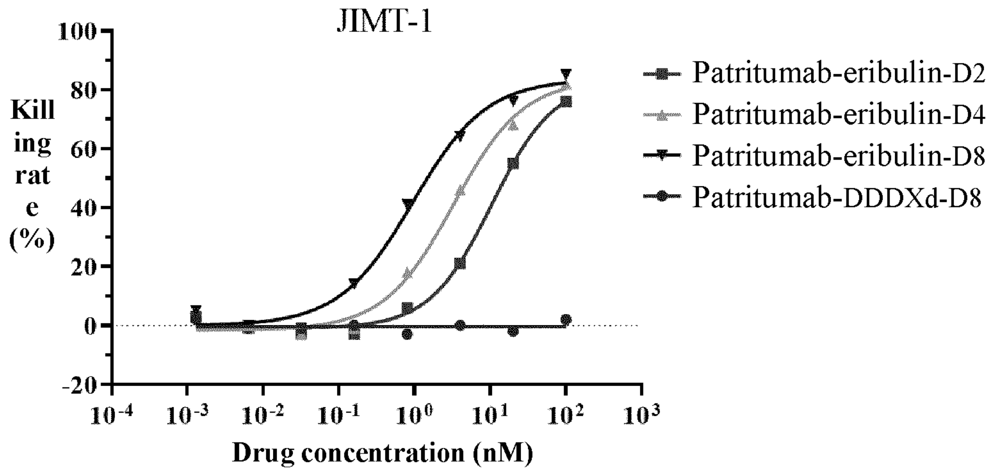


FIG. 3F

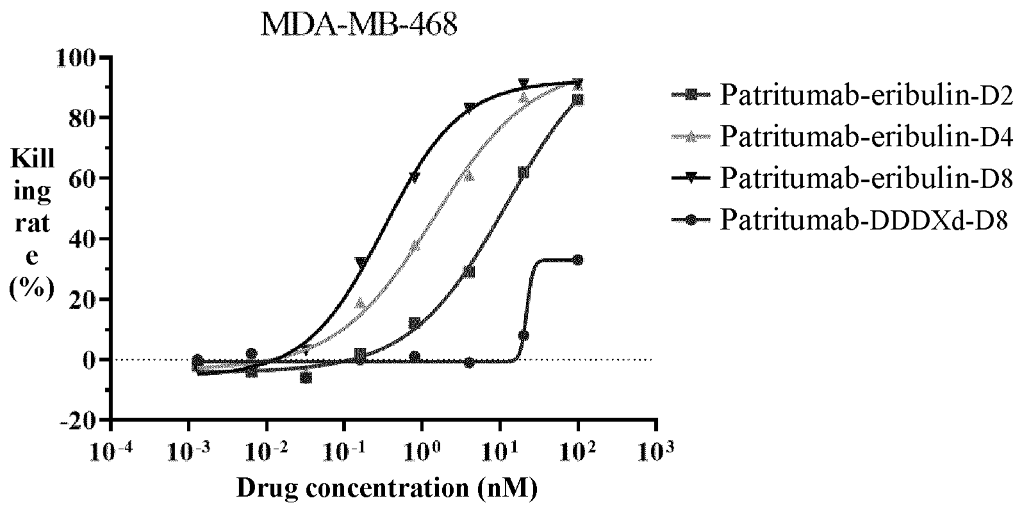


FIG. 3G

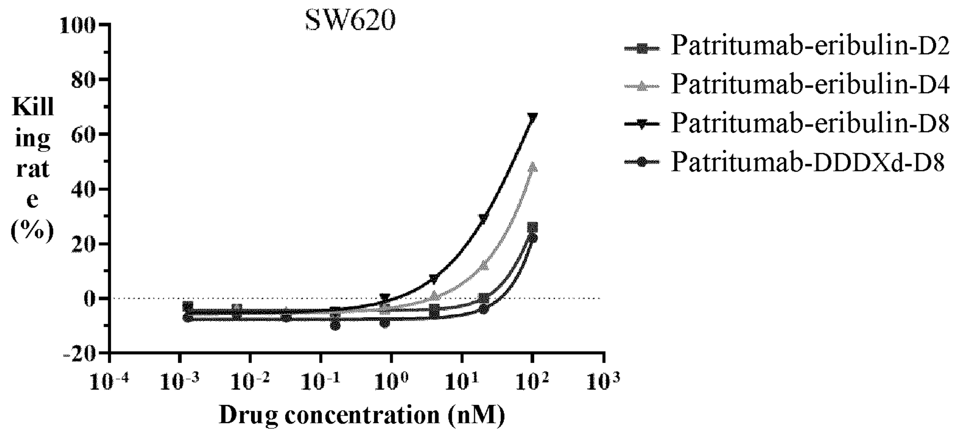


FIG. 3H

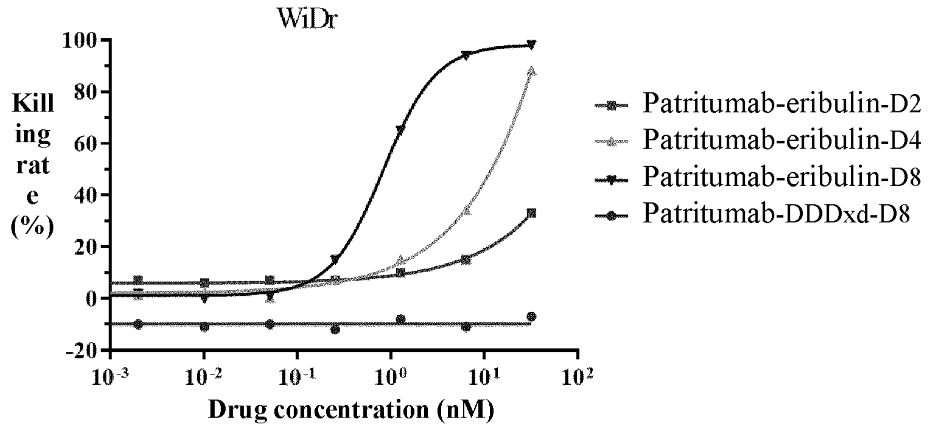


FIG. 3I

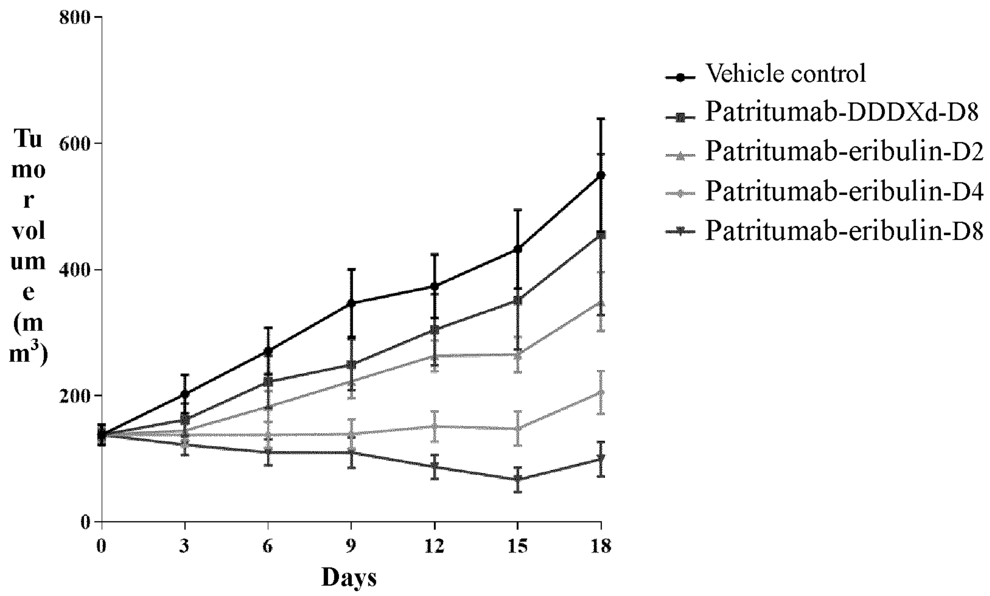


FIG. 4

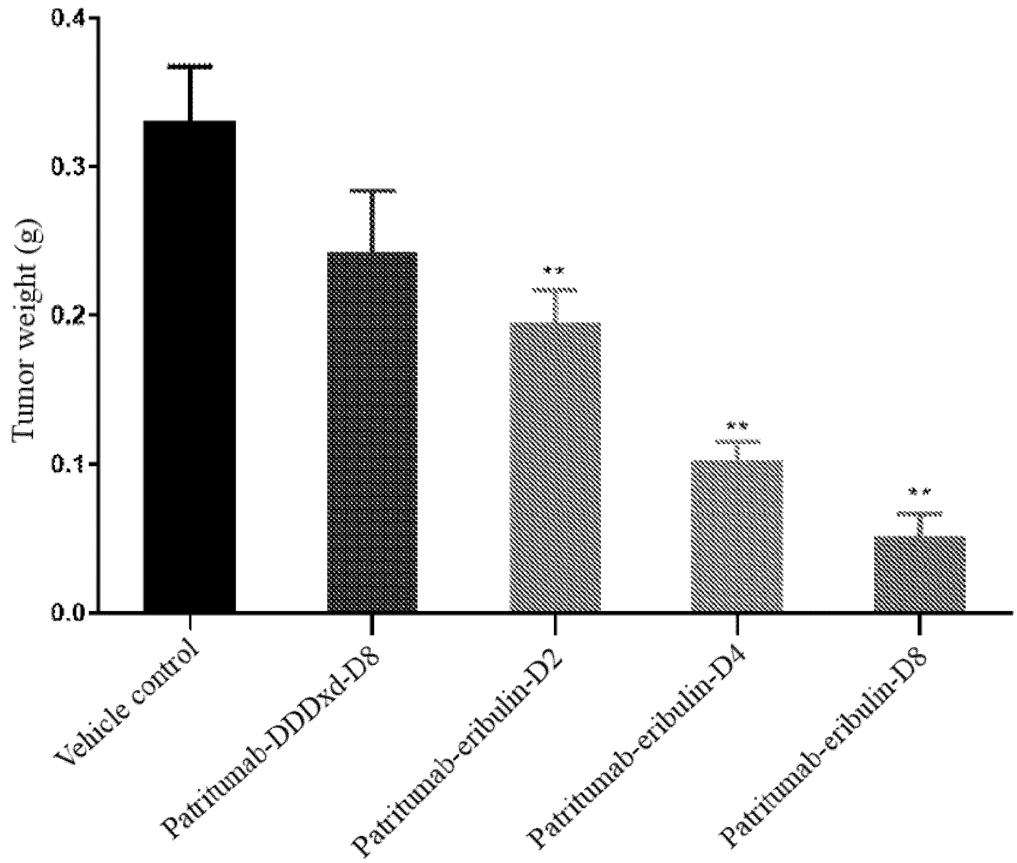
Tumor weight (g)
0.4
0.3
0.2
0.1
0.0
**
**
**
Vehicle control
Patritumab-DDDxd-D8
Patritumab-eribulin-D2
Patritumab-eribulin-D4
Patritumab-eribulin-D8

FIG. 5

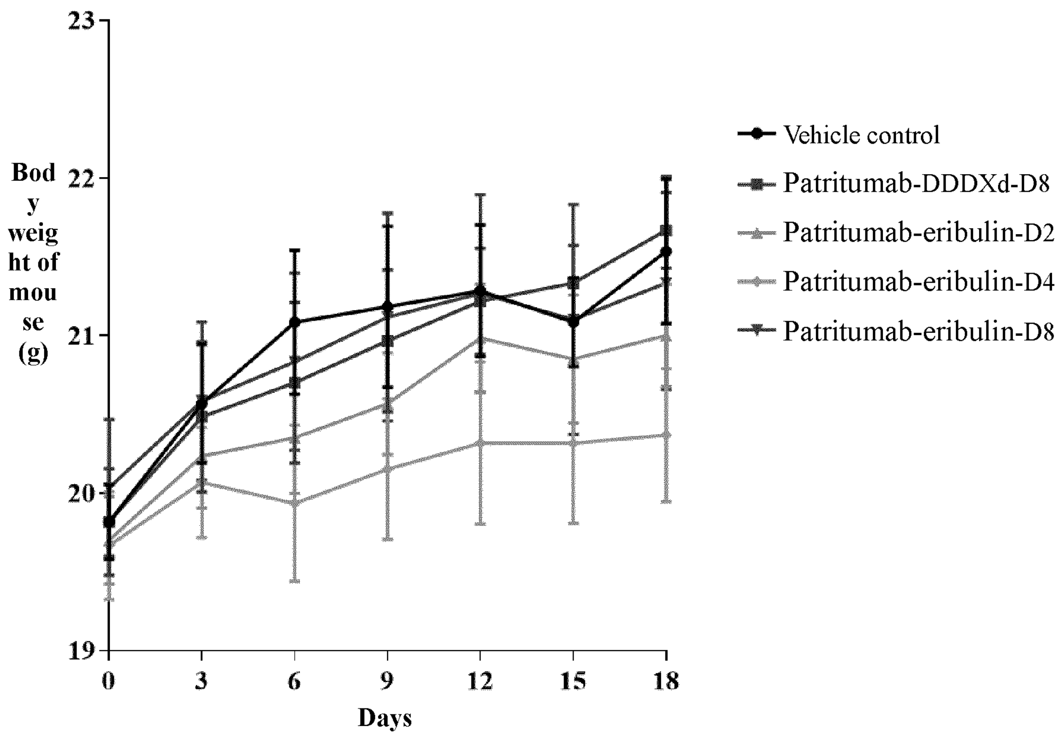
Body weight of mouse (g)
23
22
21
20
19
0
3
6
9
12
15
18
Days
Vehicle control
Patritumab-DDDXd-D8
Patritumab-eribulin-D2
Patritumab-eribulin-D4
Patritumab-eribulin-D8

FIG. 6

**INTERNATIONAL SEARCH REPORT**

International application No. **PCT/CN2022/119214**

**A. CLASSIFICATION OF SUBJECT MATTER**

A61K 47/68(2017.01)i; C07K 16/28(2006.01)i; C07D 307/20(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07K; C07D; A61P

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: 艾日布林, 抗体药物偶联物, 人类表皮生长因子受体, 抗体, 正大天晴, Eribulin, ADC, HER, antibody, CHIA TAI TIANQING

**C. DOCUMENTS CONSIDERED TO BE RELEVANT**

| Category* | Citation of document, with indication, where appropriate, of the relevant passages | Relevant to claim No. |
|---|---|---|
| PX | WO 2022022508 A1 (TUOJIE BIOTECH (SHANGHAI) CO., LTD.) 03 February 2022 (2022-02-03)<br>abstract; and embodiments 3-1 | 1, 4, 9, 13-18 |
| PX | WO 2022105878 A1 (BLISS BIOPHARMACEUTICAL (HANGZHOU) CO., LTD.) 27 May 2022 (2022-05-27)<br>abstract, and claims 1-19 | 1, 4, 9, 13-18 |
| PX | WO 2022104697 A1 (BLISS BIOPHARMACEUTICAL (HANGZHOU) CO., LTD.) 27 May 2022 (2022-05-27)<br>abstract, claims 1-10 | 1, 4, 9, 13-18 |
| X | WO 2021148003 A1 (SHANGHAI SENHUI MEDICINE CO., LTD. et al.) 29 July 2021 (2021-07-29)<br>claims 1, 27-28, and 44-46, and description, p. 74, embodiments 2-12 | 1, 4, 9, 13-18 |
| X | CN 108883198 A (EISAI INC) 23 November 2018 (2018-11-23)<br>abstract, and table 46 | 1, 4, 9, 13-18 |
| X | WO 2021132166 A1 (EISAI R&D MANAGEMENT CO., LTD.) 01 July 2021 (2021-07-01)<br>abstract, and embodiment 3 | 1, 4, 9, 13-18 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 November 2022** | **13 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/119214**

**C. DOCUMENTS CONSIDERED TO BE RELEVANT**

| Category* | Citation of document, with indication, where appropriate, of the relevant passages | Relevant to claim No. |
|---|---|---|
| Y | WO 2021148003 A1 (SHANGHAI SENHUI MEDICINE CO., LTD. et al.) 01 July 2021 (2021-07-01)<br>claims 1, 27-28, and 44-46; and description, p. 74, embodiments 2-12 | 2-3, 5-8, 10-12, 19-20 |
| Y | NAKADA, T. et al. "Novel antibody drug conjugates containing exatecan derivative-based cytotoxic payloads."<br>*Bioorganic & medicinal chemistry letters.*, Vol. 26, No. 6, 08 February 2016 (2016-02-08), pp. 1542-1545 | 2-3, 5-8, 10-12, 19-20 |
| A | CN 110997010 A (MABPLEX INTERNATIONAL LTD.) 10 April 2020 (2020-04-10)<br>entire document | 1-20 |
| A | CN 111116745 A (SHANGHAI XINLINIAN BIOPHARMACEUTICAL TECHNOLOGY CO., LTD.) 08 May 2020 (2020-05-08)<br>entire document | 1-20 |
| A | WO 2019200322 A1 (GENENTECH, INC.) 17 October 2019 (2019-10-17)<br>entire document | 1-20 |

Form PCT/ISA/210 (second sheet) (January 2015)

INTERNATIONAL SEARCH REPORT

International application No.
**PCT/CN2022/119214**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   [1] The actually submitted sequence table is an XML file of the standard ST.26.

Form PCT/ISA/210 (continuation of first sheet) (January 2015)

**INTERNATIONAL SEARCH REPORT**

International application No.
**PCT/CN2022/119214**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17-18**
because they relate to subject matter not required to be searched by this Authority, namely:

[1] Claims 17-18 set forth a method for treating cancer. The claims relate to a treatment method using a human or animal body as a direct object. Therefore, claims 17-18 do not comply with PCT Rule 39.1(iv). A search for claims 17-18 was conducted on the basis of a pharmaceutical use of the antibody-drug conjugate or a pharmaceutically acceptable salt or solvate thereof or the pharmaceutical composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/119214**

| Patent document cited in search report | Publication date (day/month/year) | Patent family member(s) | Publication date (day/month/year) |
|---|---|---|---|
| WO 2022022508 A1 | 03 February 2022 | TW 202214306 A | 16 April 2022 |
| WO 2022105878 A1 | 27 May 2022 | None | |
| WO 2022104697 A1 | 27 May 2022 | None | |
| WO 2021148003 A1 | 29 July 2021 | AU 2021210079 A1 | 25 August 2022 |
| | | BR 112022014398 A2 | 13 September 2022 |
| | | CN 114845739 A | 02 August 2022 |
| | | TW 202140078 A | 01 November 2021 |
| | | KR 20220130160 A | 26 September 2022 |
| | | CA 3168654 A1 | 29 July 2021 |
| CN 108883198 A | 23 November 2018 | UA 125024 C2 | 29 December 2021 |
| | | JP 2020019787 A | 06 February 2020 |
| | | CA 3013791 A1 | 08 September 2017 |
| | | US 2017252458 A1 | 07 September 2017 |
| | | EP 3824909 A1 | 26 May 2021 |
| | | US 2020297860 A1 | 24 September 2020 |
| | | SG 10202007520 W A | 29 September 2020 |
| | | AR 121302 A2 | 04 May 2022 |
| | | LT 3423105 T | 10 September 2021 |
| | | IL 261428 A | 31 October 2018 |
| | | CO 2018008667 A2 | 31 August 2018 |
| | | SG 11201806515 R A | 27 September 2018 |
| | | KR 20220101204 A | 19 July 2022 |
| | | BR 112018067379 A2 | 15 January 2019 |
| | | RS 62108 B1 | 31 August 2021 |
| | | HU E054726 T2 | 28 September 2021 |
| | | KR 20180115330 A | 22 October 2018 |
| | | CN 114191428 A | 18 March 2022 |
| | | CN 114191563 A | 18 March 2022 |
| | | TW 201800110 A | 01 January 2018 |
| | | JP 2020128413 A | 27 August 2020 |
| | | CL 2021000048 A1 | 28 May 2021 |
| | | MD 3423105 T2 | 31 October 2021 |
| | | ES 2880402 T3 | 24 November 2021 |
| | | HR P20211125 T1 | 15 October 2021 |
| | | PL 3423105 T3 | 25 October 2021 |
| | | JP 2019516664 A | 20 June 2019 |
| | | EP 3423105 A1 | 09 January 2019 |
| | | IL 292946 A | 01 July 2022 |
| | | US 2018193478 A1 | 12 July 2018 |
| | | WO 2017151979 A1 | 08 September 2017 |
| | | AR 107787 A1 | 06 June 2018 |
| | | SI 3423105 T1 | 31 December 2021 |
| | | PE 20181953 A1 | 17 December 2018 |
| | | PH 12018501847 A1 | 15 May 2019 |
| | | AU 2017225982 A1 | 16 August 2018 |
| | | AR 121301 A2 | 04 May 2022 |
| | | MX 2018010562 A | 20 February 2019 |
| | | DK 3423105 T3 | 26 July 2021 |
| | | KR 20220101203 A | 19 July 2022 |
| | | CL 2018002456 A1 | 21 December 2018 |

Form PCT/ISA/210 (patent family annex) (January 2015)

**INTERNATIONAL SEARCH REPORT**
**Information on patent family members**

International application No.
**PCT/CN2022/119214**

| Patent document cited in search report | Publication date (day/month/year) | Patent family member(s) | Publication date (day/month/year) |
|---|---|---|---|
| | | RU 2018134331 A | 02 April 2020 |
| | | PT 3423105 T | 19 July 2021 |
| | | RU 2021125492 A | 05 April 2022 |
| | | MA 45280 A | 09 January 2019 |
| | | CL 2021000049 A1 | 28 May 2021 |
| | | JP 2021185176 A | 09 December 2021 |
| | | CN 114272389 A | 05 April 2022 |
| WO 2021132166 A1 | 01 July 2021 | BR 112022012546 A2 | 06 September 2022 |
| | | IL 293040 A | 01 July 2022 |
| | | KR 20220120586 A | 30 August 2022 |
| | | CA 3164797 A1 | 01 July 2021 |
| | | CN 114828895 A | 29 July 2022 |
| | | AU 2020414996 A1 | 14 July 2022 |
| | | JP WO2021132166 A1 | 01 July 2021 |
| CN 110997010 A | 10 April 2020 | None | |
| CN 111116745 A | 08 May 2020 | EP 3873931 A1 | 08 September 2021 |
| | | CA 3113864 A1 | 07 May 2020 |
| | | JP 2022506310 A | 17 January 2022 |
| | | KR 20210087044 A | 09 July 2021 |
| | | WO 2020088587 A1 | 07 May 2020 |
| | | AU 2019370755 A1 | 20 May 2021 |
| | | BR 112021006919 A2 | 20 July 2021 |
| | | US 2021388082 A1 | 16 December 2021 |
| WO 2019200322 A1 | 17 October 2019 | JP 2019206514 A | 05 December 2019 |
| | | US 2019314517 A1 | 17 October 2019 |
| | | BR 112020020802 A2 | 12 January 2021 |
| | | CL 2020002624 A1 | 29 January 2021 |
| | | IL 277943 A | 30 November 2020 |
| | | KR 20210003147 A | 11 January 2021 |
| | | AR 114780 A1 | 14 October 2020 |
| | | CN 113384711 A | 14 September 2021 |
| | | EP 3773721 A1 | 17 February 2021 |
| | | CA 3095186 A1 | 17 October 2019 |
| | | CN 112040981 A | 04 December 2020 |
| | | RU 2020133722 A | 13 May 2022 |
| | | PE 20201503 A1 | 29 December 2020 |
| | | AU 2019252941 A1 | 19 November 2020 |
| | | CL 2021001956 A1 | 04 February 2022 |
| | | TW 202011994 A | 01 April 2020 |
| | | JP 2020158505 A | 01 October 2020 |
| | | CR 20200550 A | 15 December 2020 |
| | | JP 2022036932 A | 08 March 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107001463 A
- CN 109422811 A
- WO 2022033578 A1